# EUROPÄISCHE PATENTSCHRIFT

(11) **EP 1 218 040 B1**
(45) Veröffentlichungstag und Bekanntmachung des Hinweises auf die Patenterteilung: **04.05.2005**
(21) Anmeldenummer: 00964208.3
(22) Anmeldetag: 20.09.2000
(51) Int. Cl.: A61M 1/34, B01D 61/58

(54) **MEMBRANMODUL ZUR HEMODIAFILTRATION MIT INTEGRIERTER VOR- ODER NACHVERDÜNNUNG DES BLUTS**
MEMBRANE MODULE FOR THE HEMODIAFILTRATION WITH INTEGRATED PRE- OR POSTDILUTION OF THE BLOOD
MODULE A MEMBRANES POUR HEMODIAFILTRATION AVEC PREDILUTION OU POSTDILUTION INTEGREE DU SANG

(30) Priorität: 06.10.1999 DE 19947901
(43) Veröffentlichungstag der Anmeldung: 03.07.2002
(73) Patentinhaber: Membrana GmbH, 42289 Wuppertal-Barmen (DE)
(72) Erfinder: BAURMEISTER, Ulrich, 42115 Wuppertal (DE)
(74) Vertreter: Fett, Günter
(86) Internationale Anmeldenummer: PCT/EP2000/009179
(87) Internationale Veröffentlichungsnummer: WO 2001/024849

(56) Entgegenhaltungen:
- EP-A- 0 079 781
- EP-A- 0 701 826
- DE-A- 2 851 929
- US-A- 5 730 712

## Beschreibung

Die Erfindung betrifft einen Membranmodul zur Hemodiafiltration, umfassend ein zylinderförmiges Gehäuse mit einer Längserstreckung, in welchem ein in Richtung der Längserstreckung des Gehäuses orientiertes Bündel von lumenseitig durchströmbaren Hohlfasermembranen mit semipermeabler Wand angeordnet ist, deren Enden in eine erste und in eine zweite mit der Gehäuseinnenwand fluiddicht verbundene Vergussmasse so fluiddicht eingebettet sind, dass um die Hohlfasermembranen herum ein von der ersten und der zweiten Vergussmasse sowie der Gehäuseinnenwand begrenzter Außenraum ausgebildet wird, der entlang der Längserstreckung des Gehäuses durch eine jede Hohlfasermembran umschließende und zu den Hohlfasermembranen im wesentlichen quer verlaufende Trennwand aus einem im wesentlichen dimensionsstabilen Material in einen Dialysatraum und einen Substituatraum unterteilt wird, wobei der Dialysatraum und der Substituatraum jeweils zumindest eine Öffnung zur Einleitung oder zur Ausleitung eines Fluids aufweisen.

Die Hemodiafiltration ist ein membranbasiertes Kombinationsverfahren zur Blutreinigung, bei dem eine Hemodialyse und eine Hemofiltration gleichzeitig durchgeführt werden. Dieses Verfahren verbindet die Vorteile des konvektiven Stofftransports bei der Hemofiltration mit denen der Diffusion bei der Hemodialyse. Bei der Hemofiltration wird Blut an der Membran eines Hemofilters vorbeigeleitet, wobei ein Teil der Flüssigkeit des Blutes durch Ultrafiltration durch die Membran abgezogen wird. Dieser Teilstrom wird durch eine sterile und pyrogenfreie Substitutionsflüssigkeit bzw. ein Substituat ersetzt, die bzw. das entweder stromauf des Hemofilters in Form einer Vorverdünnung (Predilution) oder stromab des Hemofitters in Form einer Nachverdünnung (Postdilution) dem extrakorporalen Blutstrom zugeführt wird. Zusätzlich wird bei der Hemodiafittration noch die übliche Hemodialyse durchgeführt, bei der an der Membran des Hemodialysators Dialysierflüssigkeit bzw. Dialysat vorbeigeführt wird, so dass über die Membran hinweg eine Entfernung harnpflichtiger Substanzen erfolgen kann.

Durch die Verbindung des diffusiven Stofftransports mit dem konvektiven Stofftransport bei der Hemodiafittration lassen sich vorteilhaft nicht nur hampflichtige Stoffe mit geringem Molekulargewicht aus dem Blut entfernen. Vom konvektiven Stofftransport profitieren vor allem die langsam diffundierenden Mitteimoleküfe mit Molekulargewichten im Bereich von ca. 1 bis 55 kD, und dies um so mehr, je größer diese Moleküle sind und je größer der Filtratstrom durch die Membran ist. Bei ca. 60 kD sollen die Membranen annähernd dicht sein, so dass der Patient während einer etwa vierstündigen Behandlung nicht mehr als 4 g Proteine aus dem Blut in das Dialysat abgibt.

Beim konventionellen Hemodialyseverfahren wird nur die Flüssigkeitsmenge als Ultrafiltrat über die Dialysemembran aus dem Blut entfernt, die der Patient zwischen den Dialysebehandlungen aufgenommen hat. Die dabei entfernte Flüssigkeitsmenge entspricht etwa 6 bis 8% des Blutvoiumenstroms. Zur Durchführung von Hemodialyseverfahren werden heute in der Regel sogenannte volumenkontrollierte Dialysemaschinen eingesetzt. Diese kontrollieren die entzogene Netto-Flüssigkeitsmenge entsprechend der voreingestellten Nettofiltration über ein Bilanzieren des Dialysierflüssigkeitsstroms, der dem Dialysator zugeführt wird, mit dem Diaiysatstrom, der aus dem Dialysator abgezogen wird.

Bei der Hemodiafiltration hingegen ist demgegenüber die Menge an Ultrafiltrat aufgrund des Flüssigkeitsanteils, der zur Erhöhung des konvektiven Transports über die Membran hinweg erforderlich ist, deutlich auf etwa 20 bis 30% des Blutvolumenstroms erhöht. Dabei entspricht die dem Patienten letztendlich entzogene Netto-Flüssigkeitsmenge derjenigen bei der konventionellen Hemodialyse. Die darüber hinaus gehende Flüssigkeitsmenge zur Erhöhung des konvektiven Transports wird, wie ausgeführt, durch ein Substituat ersetzt.

Zur Durchführung von Hemodiafiltrationsverfahren werden in der Regel abgewandelte Dialysemaschinen eingesetzt, die eine Kontrolle der Ultrafiltrationsraten erlauben und ein Bilanzieren des Ultrafiltrationsvolumenstroms und des Substituatvolumenstroms vornehmen.

An die Dialysierflüssigkeit und die Substitutionsflüssigkeit werden in der Regel hinsichtlich ihrer Reinheit unterschiedliche Anforderungen gestellt. Die Dialysierflüssigkeit kann on-line aus Frischwasser und einem Elektrolytkonzentrat hergestellt werden, wobei das Frischwasser üblicherweise keimfrei und das Elektrolytkonzentrat eigensteril ist. Die Substitutionsflüssigkeit ihrerseits kann on-line aus der Dialysierflüssigkeit hergestellt werden. Jedoch ist nicht generell sichergestellt, dass die on-line hergestellte Dialysierflüssigkeit absolut steril und endotoxin- und pyrogenfrei bzw. CIS-frei ist.

Als Endotoxine werden Zellbruchstücke von abgestorbenen Bakterien bezeichnet. Die Endotoxinkonzentration wird üblicherweise mit dem sogenannten LAL-Test ermittelt, einem biologischen Assay, wie ihn beispielsweise die Firma BioWhittaker inc. herstellt. Pyrogene sind temperaturerhöhende Stoffe. Sie bewirken z.B. bei Infusion in Kaninchen eine Erhöhung der Körpertemperatur. Pyrogene können u.a. Endotoxine oder auch Exotoxine sein. Letztere werden von lebenden Bakterien produziert. Im menschlichen Blut führen diese Substanzen zur Stimulation von Monozyten, die ihrerseits Cytokine produzieren und damit eine Kaskade von weiteren Zellstimulationen auslösen. Man fasst daher heute Endotoxine, Exotoxine, Pyrogene und andere das Blut stimulierende Substanzen aus dem Dialysat unter der Abkürzung CIS (Cytokine Inducing Substances) zusammen. Eines der relevanten Cytokine, das durch Stimulation von stimulierten Monozyten produziert werden, ist Interieukin 6 (IL 6). Die Bestimmung von CIS durch den Nachweis von IL 6 ist beispielsweise bei B.L. Jaber u.a., Blood Purif. 1998, Vol. 16, Seite 210-219, beschrieben.

Daher sollte die Dialysierflüssigkeit zur Herstellung der Substitutionsflüssigkeit z.B. mittels eines Filters in den sterilen und idealerweise CIS-freien Zustand überführt werden. Natürlich lässt sich die so erzeugte Substitutionsflüssigkeit ihrerseits auch als Dialysierflüssigkeit einsetzen. Modeme Dialysemaschinen beinhalten in der Regel eine Einrichtung, mit der das Dialysat on-line derart gefiltert wird, dass es eine Konzentration von Endotoxinen von weniger als 0,5 EU pro ml Dialysat aufweist. Damit treten auch bei der sogenannten High-fiux Dialyse nahezu keine pyrogenen Reaktionen beim Patienten mehr auf, die bei durch Endotoxine verunreinigtem Dialysat häufig beobachtet werden. Allerdings kann bei einer Endotoxinkonzentration von < 0,03 EU/ml, der Nachweisgrenze der gängigen LAL-Tests, noch CIS im Dialysat sein. Die Forderung nach CIS-freiem Dialysat ist also schärfer als die nach LAL-negativem Dialysat.

In der EP-A 692 269 wird eine Hemodiafiltrationsvorrichtung beschrieben, welche zwei in Reihe geschaltete Blutfilter aufweist. Die Blutfilter enthalten jeweils Membranen, die an ihrer einen Seite vom zu reinigenden Blut überströmt werden und an ihrer anderen Seite von Dialysierflüssigkeit. Die der Hemodiafiltrationsvorrichtung zugeführte Dialysierflüssigkeit wurde zuvor über einen Sterilfilter geleitet. In der in der EP-A 692 269 beschriebenen Vorrichtung erfolgt in einem der beiden Blutfilter aufgrund des dort eingestellten positiven Transmembrandrucks in Richtung des Blutweges über die Membran dieses Blutfilters ein Übergang von Dialysierflüssigkeit als Substitutionsflüssigkeit direkt in das Blut. In dem zweiten Blutfilter wird ein negativer Transmembrandruck erzeugt, und es erfolgt dort über eine Diafiltration eine Abtrennung eines Teils der Blutflüssigkeit und eine Entfernung harnpflichtiger Substanzen in das Dialysat.

Derartige Hemodiafiltrationsvorrichtungen mit in Reihe geschalteten Blutfiltern erweisen sich im Betrieb als aufwendig und lassen sich aufgrund der Konzeption und der damit verbundenen speziellen und aufwendigen Steuerung auf den marktüblichen Dialysemaschinen in der Regel nicht einsetzen.

Auch die EP-A 451 429 offenbart eine Hemodiafiltrationsvorrichtung, welche zwei in Reihe geschaltete Membranmodule enthält. Hierbei ist der erste Membranmodul ein Hernofilter, in dem über Ultrafiltration dem zu reinigenden Blut ein Teilstrom von Flüssigkeit entzogen wird, der vornehmlich die aus dem Blut zu entfernenden mittelmolekularen Substanzen enthält. Das Ultrafiltrat wird in einem speziellen Filter regeneriert und dem Blutstrom wieder zugeführt, bevor dieser in den zweiten Membranmodul eingeleitet wird. Dieser Blutstrom wird dann im zweiten Membranmodul einer Hemodialyse unterzogen.

Zu den zuvor genannten Nachteilen in Reihe geschalteter und getrennter Blutfilter tritt für die in der EP-A 451 429 beschriebenen Hemodiafiltrationsvorrichtungen als weiterer Nachteil hinzu, dass ein spezieller Regenerator erforderlich ist, mittels dessen das Ultrafiltrat gereinigt werden muss.

In der DE-A 196 07 162 wird ein Hemodiafiltrationssystem mit einer gesteuerten Zuführung für ein Substituat und einer gesteuerten Zuführung für ein Dialysat in einen Dialysator beschrieben, wobei der Dialysator als einheitliches Bauteil für die Blutbehandlung, die Substituatfiltrierung und die Vermischung des Substituats mit dem zu behandelnden Blut ausgebildet ist. Der Dialysator enthält in seinem langgestreckten Gehäuse zwei nebeneinander angeordnete Membranmodule mit jeweils einem Bündel von Hohlfasermembranen, wobei die Membranmodule durch eine zu den Hohlfasermembranen im wesentlichen parallele Trennwand voneinander getrennt sind. Der erste Membranmodul wird zur Hemodiafiltration eingesetzt und der zweite Membranmodul zur Steriffiltration des Substituats. Der Dialysator umfasst des Weiteren eine Kammer, in der das gereinigte Substituat mit dem zu behandelnden Blut vereinigt wird.

Zwar ist das in der DE-A 196 07 162 beschriebene Hemodiafiltrationssystem im Vergleich zu den Systemen mit in Reihe geschalteten mehreren Blutfiltern einfacher und übersichtlicher aufgebaut. Jedoch erweist sich die Herstellung der in der DE-A 196 07 162 offenbarten, zwei Module enthaltenden Dialysatoren insbesondere auch wegen der Handhabung zweier unterschiedlicher Hohlfasermembranbündel als schwierig. Darüber hinaus sind die Membranmodule im Dialysator nicht rotationssymmetrisch angeordnet, so dass die Gefahr einer ungleichförmigen Durchströmung insbesondere des Außenraums um die Hohlfasermembranen des ersten Membranmoduls besteht, der zur Hemodiafiltration eingesetzt wird. Ferner ist auch bei Dialysatoren, wie sie in der DE-A 196 07 162 beschrieben werden, eine zusätzliche Pumpeneinrichtung für den Transport des Substituats erforderlich, die in heute üblichen Dialysemaschinen nicht vorhanden ist.

US-A 5 730 712 offenbart einen Modul zur Hemodiafiltration mit einer axialen Trennwand, die zu den Hohlfasermembranen parallel verläuft. Durch die axiale Trennwand entstehen zwei Dialysaträume, die durch eine Öffnung in der Trennwand hydraulisch miteinander verbunden sind. Die Hohlfasem werden dadurch in zwei Bündel unterteilt. Im ersten Dialysatraum ist der Druck der Dialysierflüssigkeit höher als der Druck des Blutes, das durch die Hohlfasermembranen strömt, so dass die Dialysiertlüssigkeit durch die Wand der Hohlfasermembranen in den Blutstrom eintritt. Im zweiten Dialysatraum wird dem Blut Ultrafiltrat entzogen, das mit der Dialysierflüssigkeit über den Dialysatauslass das Modul verlässt. Durch das Aufteilen der Hohlfasermembranen in zwei Bündel wird auch der Blutstrom in zwei Teilströme geteilt, wodurch dann auch nur aus einem Teilstrom des Blutes hampflichtige Substanzen eliminiert werden können.

Die EP-A 701 826 offenbart einen Hemodiafilter, der ein einzelnes Bündel von Hohlfasermembranen zur Blutbehandlung, zur Filtrierung des Substituats und zur Zuführung des Substituats zum Blut enthält. Bei diesem Hemodiafilter befindet sich im Außenraum des Hemodiafilters um die Hohlfasermembranen herum ein Material, welches beim Einsatz dieses Hemodiafilters zur Hemodiafiltration durch die Dialysierflüssigkeit in seinen Dimensionen verändert, d. h. gequollen wird und so zu einer Verengung führt. Hierdurch wird ein Druckabfall für die durch den Außenraum strömende Dialysierflüssigkeit zwischen der stromaufwärts und der stromabwärts gelegenen Seite der Verengung erzeugt. Als durch die Dialysierflüssigkeit quellbare Materialien werden verschiedene Copolymere aufgezählt, die auf die Hohlfasermembranen an der gewünschten Stelle aufgetragen werden, oder quellbare faserförmige Materialien, die z.B. mit den Hohlfasermembranen verwoben werden.

Die Einbringung des quellbaren Materials ist zum einen aufwendig, zum anderen ist auch eine genaue, stabile und reproduzierbare Positionierung über den Bündelquerschnitt hinweg nicht gewährleistet und in der Praxis nicht umsetzbar. Auch sind hinsichtlich der verwendbaren quellbaren Materialien enge Grenzen gesetzt, da der Einsatz des Hemodiafilters zur Blutreinigung wegen des Kontakts mit körpereigenen Flüssigkeiten eine hohe Blutverträglichkeit der eingesetzten Materialien erfordert. Da gemäß der EP-A 701 826 der Quellgrad des eingebrachten quellbaren Materials von der verwendeten Dialysierflüssigkeit abhängt, ist der Quellgrad schlecht zu kontrollieren und zu reproduzieren. Die Wirkung der durch dieses Material erzeugten Verengung auf den Dialysatfluss und den Substituatfluss ist nicht im Voraus bestimmbar oder erfordert zumindest umfangreiche Vorversuche und aufwendige Abschätzungen. Aufgrund der Quellung ist darüber hinaus eine Abhängigkeit der Dimensionen des Materials und damit der Querschnittsverengung vom anliegenden Differenzdruck gegeben, wobei es im ungünstigen Fall bei einer Erhöhung der Druckdifferenz auch zu einem völligen Verschluss kommen kann.

Aus den geschilderten Nachteilen ist leicht ableitbar, dass für die in der EP-A 701 826 offenbarten Hemodiafilter in der Anwendung keine kontrollierbaren und reproduzierbaren Strömungsverhältnisse vorliegen.

Es ist daher Aufgabe der vorliegenden Erfindung, einen Membranmodul für die Hemodiafiltration zur Verfügung zu stellen, der einen einfachen und kompakten Aufbau aufweist, bei welchem eine verbesserte Vorausbestimmbarkeit und Reproduzierbarkeit der Zuführung von Substituat und Dialysat vorliegen und weicher in volumenkontrollierte Dialysemaschinen ohne größere Änderungen eingesetzt werden kann.

Die Aufgabe wird durch einen Membranmodul zur Hemodiafiltration gelöst, welcher ein zylinderförmiges Gehäuse mit einer Längserstreckung umfasst, in welchem ein in Richtung der Längserstreckung des Gehäuses orientiertes Bündel von lumenseitig durchströmbaren Hohlfasermembranen mit semipermeabler Wand angeordnet ist, deren Enden in eine erste und in eine zweite mit der Gehäuseinnenwand fluiddicht verbundene Vergussmasse so fluiddicht eingebettet sind, dass um die Hohlfasermembranen herum ein von der ersten und der zweiten Vergussmasse sowie der Gehäuseinnenwand begrenzter Außenraum ausgebildet wird, der entlang der Längserstreckung des Gehäuses durch eine jede Hohtfasermembran umschließende und zu den Hohlfasermembranen im wesentlichen quer verlaufende Trennwand aus einem im wesentlichen dimensionsstabilen Material in einen Dialysatraum und einen Substituatraum unterteilt wird, wobei der Dialysatraum und der Substituatraum jeweils zumindest eine Öffnung zur Einleitung bzw. zur Ausleitung eines Fluids aufweisen, dadurch gekennzeichnet, dass im Außenraum mindestens eine Drossel angeordnet ist, über die der Dialysatraum und der Substituatraum miteinander in Fluidverbindung stehen.

Im Rahmen der vorliegenden Erfindung wird unter einer Drossel eine definierte Verengung eines Strömungsquerschnitts zur gezielten Erzeugung eines definierten Druckverlustes bei der Strömung eines Fluids durch diese Verengung verstanden, d.h. die Drossel weist einen in Strömungsrichtung des Fluids gesehen gegenüber dem Strömungsquerschnitt vor und hinter der Drossel reduzierten Strömungsquerschnitt auf. Dabei besitzt der Strömungsquerschnitt der Drossel einen definierten, vom durchströmenden Fluid unabhängigen festen Wert oder ist auf einen definierten, vom durchströmenden Fluid unabhängigen Wert einstellbar. Bei derartigen Drosseln ist der bei der Durchströmung entstehende Druckverlust vorausbestimmbar. Zu den Drosseln mit festem bzw. auf einen festen Wert einstellbaren Strömungsquerschnitt zählen beispielsweise Blenden in Form von Lochblenden oder Spaltblenden oder auch Kapillarröhrchen mit definierten Durchmessern. In einer bevorzugten Ausführungsform des erfindungsgemäßen Membranmoduls ist die Drossel einstellbar.

Unter einem im wesentlichen dimensionsstabilen Material wird im Rahmen der vorliegenden Erfindung ein Material verstanden, weiches in der Anwendung des erfindungsgemäßen Hemodiafiltrationsmoduls unter den dann vorherrschenden Bedingungen seine Dimension und seine Form gegenüber dem Ursprungszustand des Moduls nach seiner Herstellung im wesentlichen beibehält und insbesondere durch die dabei eingesetzten Flüssigkeiten, d.h. vor allem die Dialysierflüssigkeit, nicht gequollen wird.

In einer bevorzugten Ausführungsform des erfindungsgemäßen Membranmoduls ist das Gehäuse um seine in Richtung der Längserstreckung orientierte Längsachse kreiszylinderförmig und die Hohtfasermembranen sind in einem Bündel angeordnet, welches um die Längsachse im wesentlichen rotationssymmetrisch ist.

Bei dem erfindungsgemäßen Hemodiafilter sind die Hohlfasermembranen an ihren Enden jeweils in Vergussmassen eingebettet, die gleichzeitig den um die Hohlfasermembranen ausgebildeten Außenraum gegenüber einem Verteilerraum, in welchem das über eine Bluteinlasseinrichtung in den Sammelraum eingeleitete und zu behandelnde Blut auf die Lumina der Hohtfasermembranen verteilt wird, und gegenüber einem Sammelraum, in dem das aus den Lumina ausströmende Blut gesammelt und über eine Blutausiasseinrichtung aus dem Modul ausgeleitet wird, verschließen. Dabei erstrecken sich die Hohlfasermembranen mit ihren stirnseitig offenen Enden durch die jeweilige Vergussmasse hindurch und stehen mit dem Verteilerraum bzw. dem Sammelraum lumenseitig in Verbindung, so dass sie von dem zu behandelnden Blut durchströmbar sind.

Aufgrund dessen, dass bei dem erfindungsgemäßen Hemodiafilter dieselben Hohlfasermembranen zur Blutbehandlung, zur Filtrierung des Substituats und zur Zuführung des Substituats zum Blut dienen und der Dialysatraum und der Substituatraum mittels einer Trennwand voneinander getrennt sind, ergibt sich, dass sich in Erstrekkungsrichtung der Hohlfasermembranen gesehen Dialysatraum und Substituatraum an unterschiedlichen Positionen entlang der Hohtfasermembranen nebeneinander angeordnet sind, wobei die Trennwand den Innenquerschnitt des Gehäuses zumindest weitgehend ausfüllt. Dabei ist bei dem erfindungsgemäßen Membranmodul erfindungsgemäß im Außenraum mindestens eine Drossel angeordnet, über die der Substituatraum und der Dialysatraum miteinander in Fluidverbindung stehen. Daraus folgt, dass die mindestens eine Drossel in der Trennwand oder in deren Bereich, d.h. in die Trennwand integriert oder die Trennwand innerhalb des Gehäuses im Außenraum umgehend angeordnet ist.

In einer vorteilhaften Ausführungsform weist der erfindungsgemäße Membranmodul im Bereich des Substituatraums allein eine Öffnung auf, die als Einlasseinrichtung zur Einleitung der Dialysierflüssigkeit in den Substituatraum dient, und im Bereich des Dialysatraums allein eine Öffnung, die als Auslasseinrichtung zur Ausweitung des Dialysats aus dem Dialysatraum dient. Dies ist besonders günstig in Bezug auf eine Kompatibilität mit den Anschlussmöglichkeiten moderner Dialysemaschinen.

In der Anwendung des erfindungsgemäßen Hemodiafilters wird das zu reinigende Blut durch das Lumen der Hohlfasermembranen geleitet. Über geeignete Fördermittel, also z.B. über eine Pumpe, wird dem Hemodiafilter ein Dialysierflüssigkeitsstrom zugeführt und über die als Einlasseinrichtung für die Dialysierflüssigkeit dienende Öffnung des Substituatraums, d.h. über die Einlasseinrichtung des Substituatraums, mit Überdruck in den Substituatraum eingeleitet. Ein Teil der in den Substituatraum eingeleiteten Dialysierflüssigkeit wird über die Wände der im Substituatraum befindlichen Abschnitte der Hohlfasermembranen dem durch die Lumina der Hohlfasermembranen strömenden Blut als Substituat mit einem definierten Volumenstrom zugeführt. Der Substituatvolumenstrom wird dabei maßgeblich durch den Überdruck und die Durchlässigkeit der Hohtfasermembranen beeinflusst. Die Menge an pro Zeiteinheit zugeführtem Substituat, d.h. der Substituatvolumenstrom ergibt sich aus der Differenz des im Bereich des Dialysatraums über Ultrafiltration aus dem Blut entzogenen Flüssigkeitsstroms und der von der Dialysemaschine kontrollierten und fest voreingestellten Nettofiltration.

Über die erfindungsgemäß eingesetzte mindestens eine im Außenraum angeordnete Drossel wird der größere Teil der insgesamt dem Hemodiafilter zugeführten Dialysierflüssigkeit als Dialysat mit definiertem Volumenstrom in den Dialysatraum geleitet und durchströmt diesen. Der Volumenstrom des in den Dialysatraum eingeleiteten Dialysats und damit das Verhältnis des Diafysatvoiumenstroms zum Substituatvolumenstrom resultiert im wesentlichen aus der Anzahl der Drosseln sowie dem durch diese erzeugten Strömungswiderstand im Verhältnis zu dem Strömungswiderstand, der vom Substituatstrom beim Permeieren durch die Wand der Hohlfasermembranen überwunden werden muss.

Im Dialysatraum wird das Dialysat an den Hohtfasermembranen vorbeigeleitet, wobei es dort gleichzeitig das über Ultrafiltration durch die Wände der Hohlfasermembranen aus dem Blut abgezogene Ultrafiltrat aufnimmt, so dass über konvektive und diffusive Transportmechanismen dem Blut die harnpflichtigen Stoffe entzogen werden. Das mit dem Ultrafiltrat vermischte Dialysat wird über die als Auslasseinrichtung für das Dialysat dienende Öffnung des Dialysatraums, d.h. über die Auslasseinrichtung des Dialysatraums, aus dem Dialysatraum abgezogen. Je nach Durchströmungsrichtung des Bluts durch die Hohtfasermembranen kann das Substituat dem Blut zugeführt werden, bevor dieses der Hemodiafittration unterzogen wird (Vorverdünnung) oder nachdem dieses der Hemodiafiltration unterzogen wurde (Nachverdünnung). Der erfindungsgemäße Membranmodul ist damit so ausgelegt, dass eine Hemodiafiltration mit integrierter Vor- oder Nachverdünnung des Bluts durchgeführt werden kann.

Durch die erfindungsgemäße Ausgestaltung des erfindungsgemäßen Membranmoduls zur Hemodiafiltration d.h. des erfindungsgemäßen Hemodiafilters ist es möglich, auf einfache, kontrollierbare und reproduzierbare Weise die bei der Hemodiafiltration erforderliche Verdünnung des Bluts mit Substituat und die Hemodiafiltration in einem einzigen Membranmodul zu integrieren, welcher wie übliche Hemodialysatoren alleine ein Bündel von Hohlfasermembranen enthält, so dass die Hemodiafittration in auf dem Markt befindlichen Dialysemaschinen mit volumenstromkontrollierter Ultrafiltration durchführbar ist. Gleichzeitig gewährleistet die Trennung des Substituatraums vom Dialysatraum mittels der erfindungsgemäßen, im wesentlichen dimensionsstabilen und in dem Dialysat im wesentlichen nicht-quellbaren Trennwand in Verbindung mit der mindestens einen erfindungsgemäßen Drossel, über die der Dialysatraum und der Substituatraum in Fluidverbindung stehen, eine gut vorausbestimmbare, und reproduzierbare Einstellung des Volumenstromverhältnisses von Substituat und Dialysat.

Gegenüber bekannten Hemodiafiltem, wie sie z.B. in der DE-A 196 07 162 oder der EP-A 701 826 beschrieben sind, bietet der erfindungsgemäße Hemodiafilter mit einer Trennwand, in oder in deren Bereich mindestens eine Drossel angeordnet ist, den Vorteil einer sehr kompakten Bauweise mit nur einem einzigen Hohlfasermembranbündel, welches zur Blutbehandlung, zur Filtrierung des Substituats und zur Zuführung des Substituats zum Blut dient, wobei über die Drossel eine verlässliche und genaue Einstellung der Druck- bzw. Druckverlustverhältnisse erfolgt, aus denen definierte Volumenströme von Dialysat und Substituat resultieren.

Die DE-A 28 51 929 offenbart eine Modulkonstruktion auf Basis von Hohlfasermembranen, bei der der Dialysatraum durch eine dichte Trennwand in zwei Teilräume unterteilt wird. In einer Ausführungsform wird durch den einen Teilraum, der mit einer Einlasseinrichtung und mit einer Auslasseinrichtung versehen ist, Dialysat hindurchgeleitet, um über Diffusion hampflichtige Substanzen aus dem durch die Hohlfasermembranen strömenden Blut zu entfernen. An den zweiten Teilraum, welcher mit einer Auslasseinrichtung versehen ist, wird ein Unterdruck angelegt, um aus dem die Hohlfasermembranen durchströmenden Blut über die Wände der Hohlfasermembranen ein Filtrat abzuziehen. Bei der DE-A 28 51 929 schließt die Trennwand die beiden Teilräume des Moduls gegeneinander fluiddicht ab und enthält keine Drossel, über die eine Überleitung von Dialysierflüssigkeit aus dem einen Teilraum in den anderen möglich wäre. Damit ist dieser Modul aber für einen Einsatz bei der Hemodiafiltration nicht geeignet.

Bei dem erfindungsgemäßen Hemodiafiltrationssystem kann die Zuführung des Substituats zum Blut erfolgen, bevor oder nachdem das Blut der Diafiltration im Bereich des Dialysatraums unterworfen wurde. Im Einzelfall ist es auch möglich, den Substituatraum und damit die Zuführung des Substituats zum Blut entlang der Erstreckung der Hohtfasermembranen aufzuteilen und einen Teil des Substituats vor und einen Teil nach der Diafiltration dem Blut zuzuführen. In diesem Fall sind in dem erfindungsgemäßen Hemodiafilter entlang der Erstreckung der Hohlfasermembranen benachbart zu den Einbettungen der Hohliasermembranenden zwei Substituatteilräume angeordnet, die jeweils über eine Trennwand von einem dazwischenliegenden Dialysatraum getrennt sind, wobei in oder im Bereich mindestens einer der Trennwände mindestens eine Drossel angeordnet ist. Entsprechend ist es auch möglich, den Dialysatraum und damit die Diafiltration aufzuteilen und im Hemodiafilter entlang der Erstreckung der Hohtfasermembranen benachbart zu den Einbettungen der Hohlfasermembranenden zwei Dialysatteilräume anzuordnen, die von einem dazwischenliegenden Substituatraum jeweils über eine Trennwand getrennt sind. In diesen Fällen ist in oder im Bereich jeder der Trennwände mindestens eine Drossel angeordnet.

Gemäß einer bevorzugten Ausgestaltung des erfindungsgemäßen Membranmoduls ist die Trennwand fluiddicht mit der Gehäuseinnenwand verbunden und die mindestens eine im Außenraum angeordnete Drossel in die Trennwand integriert, so dass die Trennwand über die mindestens eine Drossel permeabel für die Dialysierflüssigkeit ist und ein Teil der Dialysierflüssigkeit als Dialysat aus dem Substituatraum in den Dialysatraum geleitet werden kann.

Im einfachsten Fall handelt es sich bei der mindestens einen in die Trennwand integrierten Drossel um mindestens ein Loch mit definiertem Durchmesser, in der Regel um eine Vielzahl von Löchern, die vorzugsweise in einem Ring um das Hohlfasermembranbündel herum angeordnet sind. Hierbei sind die Einlasseinrichtung des Substituatraums und die Auslasseinrichtung des Dialysatraums zweckmäßigerweise an den der Trennwand abgewandten Enden der jeweiligen Räume angeordnet.

Vorzugsweise ist die in die Trennwand integrierte mindestens eine Drossel mindestens eine in die Trennwand eingebrachte Kapillare, über deren Lumen der Dialysatraum und der Substituatraum miteinander in Fluidverbindung stehen. Besonders bevorzugt sind mehrere Kapillaren um das Bündel der Hohlfasermembranen herum angeordnet, wobei eine besonders vorteilhafte Anordnung eine ringförmige Anordnung ist, beispielsweise bei einem Bündel mit im wesentlichen kreisförmigem Querschnitt in Form eines Kreisrings.

Über den Durchmesser des Lumens der Kapillaren, die Anzahl der Kapillaren und ihre Länge läßt sich unter Zuhilfenahme einfacher strömungstechnischer Berechnungen die Drosselwirkung ermitteln und damit das Verhältnis von Dialysatvolumenstrom und Substituatvolumenstrom vorausbestimmen und entsprechend voreinstellen.

Hinsichtlich der Kapillariänge lassen sich zwei bevorzugte Ausführungsformen unterscheiden. Bei Verwendung kurzer Kapillaren ist es für eine problemlose Einbringung derartiger Kapillaren in die Trennwand zweckmäßig, wenn die Kapillarlänge größer ist als die Dicke der Trennwand. Jedoch sollten im Hinblick auf eine gleichmäßige Durchströmung des Dialysatraums die Kapillaren nur wenig in diesen hineinragen. Vorzugsweise übersteigt die Länge der Kapillaren die Dicke der Trennwand um weniger als 100%. Für diese Fälle sind die Einlasseinrichtung des Substituatraums und die Auslasseinrichtung des Dialysatraums zweckmäßigerweise an den der Trennwand abgewandten Enden der jeweiligen Räume angeordnet.

In einer weiteren bevorzugten Ausführungsform erstreckt sich mindestens eine lange Kapillare durch den Dialysatraum hindurch und endet im Bereich vor der den Dialysatraum begrenzenden Vergussmasse, wobei dann die Auslasseinrichtung des Dialysatraums zur Trennwand benachbart angeordnet ist. Diese Ausführungsform des erfindungsgemäßen Membranmoduls lässt sich vorzugsweise für eine Reinigung von Blut einsetzen, bei der eine Vorverdünnung des Bluts mit Substituat erfolgt, bevor dieses der Diafiltration unterzogen wird. Das Blut strömt dann an der Seite des Gehäuses in den Hemodiafilter ein, die dem Substituatraum zugewandt ist und tritt am entgegengesetzten, dem Dialysatraum zugewandten Ende aus dem Gehäuse aus. Auf seinem Weg durch die Hohlfasermembranen wird das Blut zunächst mit Substituat verdünnt und anschließend im Bereich des Dialysatraums der Hemodiafiltration unterzogen, wobei aufgrund der Erstreckung der als Drossel eingesetzten mindestens einen langen Kapillaren durch den Dialysatraum hindurch und der Anordnung der Dialysatraumöffnung in der Nähe der Trennwand das Dialysat entgegengesetzt zur Strömungsrichtung des Bluts an der Außenseite der Hohlfasermembranen vorbeigeführt wird. Bei Verwendung mehrerer Kapillaren können diese bei dieser Ausführungsform z.B. auch mit den Hohtfasermembranen gemischt werden und an ihrem einen Ende mit diesen zusammen in die Trennwand eingebettet werden, wobei die Trennwand in diesem Fall aus einer Vergussmasse hergestellt wird. Hierdurch wird in der Anwendung eine gleichmäßige Dialysatverteilung erreicht.

In einer besonders bevorzugten Ausführungsform ist in die Trennwand eine einzelne, sich durch den Dialysatraum hindurch erstreckende lange Kapillare eingebracht, die zentral im Bündel der Hohlfasermembranen angeordnet ist und über die der Substituatraum und der Dialysatraum miteinander in Fluidverbindung stehen. Zur Erzielung eines erforderlichen Durchsatzes an Dialysierflüssigkeit durch diese als Drossel fungierende Kapillare kann diese auch einen relativ großen Durchmesser aufweisen. Es ist auch möglich, dass die Kapillare eher den Durchmesser eines Rohres hat, d.h. als Rohr vorliegt, wobei dann z.B. die gewünschte Drosselwirkung dieses Rohres durch gezielte Verengungen im Innenraum des Rohres erzeugt werden kann. Derartige Rohre können vorteilhaft gleichzeitig als Wickelkern dienen, wenn die Hohlfasermembranen nebeneinander und zueinander parallel in Matten angeordnet sind und das Hohlfasermembranbündel beispielsweise durch spiralförmiges Wickeln mindestens einer Hohlfasermembranmatte um das Rohr hergestellt wird, wobei die Hohlfasermembranen parallel zur Rohrachse angeordnet sind. Dabei kann sich das Rohr auch über die gesamte Länge zwischen der ersten und der zweiten Vergussmasse erstrecken und in diese Vergussmassen jeweils so eingebettet sein, dass sein Innenraum gegenüber dem Verteilerraum bzw. dem Sammelraum fluiddicht abgetrennt ist. In diesem Fall weist es im Bereich des Substituatraums und im Bereich des Dialysatraums Öffnungen in seiner Wand auf, über die im Bereich des Substituatraums Diatysierftüssigkeit in das Rohr eintreten und im Bereich des Dialysatraums als Dialysat austreten kann. Die Öffnungen können gleichzeitig auch als Drossel fungieren.

In einer weiteren vorteilhaften Ausführungsform des erfindungsgemäßen Membranmoduls ist die mindestens eine Drossel mindestens eine in die Trennwand eingebettete semipermeable, vorzugsweise durchgehend mikroporöse Kapillarmembran, deren Lumen an ihrem einen Ende offen und an ihrem anderen Ende geschlossen ist. Bevorzugt ist diese mindestens eine Kapillarmembran so in die Trennwand eingebettet, dass sie mit ihrem geschlossenen Ende in den Substituatraum hineinragt und ihr offenes Ende in den Dialysatraum mündet oder in diesen hineinragt. Bei der Hemodiafiltration strömt dann Dialysierflüssigkeit im Bereich des Substituatraums im sogenannten dead-end Modus über die semipermeable Wand der mindestens einen Kapillarmembran in deren Lumen ein und strömt über das geöffnete Ende der Kapillarmembran in den Dialysatraum aus. Die Drosselwirkung der mindestens einen Kapillarmembran ergibt sich aus dem bei der Durchströmung der porösen semipermeablen Kapillarmembranwand entstehenden Druckverlust.

In einer ebenfalls vorteilhaften Ausgestaltung des erfindungsgemäßen Membranmoduls sind mehrere Drosseln in Form von Ringspaltsegmenten in der Trennwand um das Bündel der Hohlfasermembranen herum oder an der Trennwand an deren äußerem Umfang angeordnet.

Gemäß einer weiteren bevorzugten Ausführungsform des erfindungsgemäßen Membranmoduls ist die mindestens eine im Außenraum angeordnete Drossel ein Ringspalt, welcher um das Bündel der Hohlfasermembranen herum zwischen der Trennwand und der Gehäuseinnenwand ausgebildet ist, wobei in diesem Fall die Trennwand natürlich nicht fluiddicht mit der Gehäuseinnenwand verbunden ist. Zur Fixierung der Trennwand und zur Stabilisierung des Abstands sind zwischen Trennwand und Gehäuseinnenwand zweckmäßigerweise Abstandhalter eingebracht, so dass für einen definierten stabilen Ringspalt und damit für eine definierte Drosselwirkung Sorge getragen ist.

Es ist von Vorteil, wenn die Trennwand zusammen mit dem Hohlfasermembranbündel an ihrem äußeren Umfang fluiddicht in eine Hülse eingebettet ist. Diese Hülse weist dann gegenüber der Gehäuseinnenwand einen definierten Abstand auf, wodurch ein die Drossel ausbildender Ringspalt ausgeformt wird. Durch die Verwendung einer Hülse kann außer über die Spaltbreite auch über die Länge der Hülse in weiten Bereichen Einfluss auf den durch den Spalt erzeugten Druckvertust und damit auf die Drosselwirkung genommen werden. Die erforderliche Länge des im Bereich der Trennwand zwischen Hülse und Gehäuseinnenwand ausgebildeten Ringspalts entlang der Längserstreckung des Gehäuses resultiert dann zusammen mit der Ringspaltbreite aus der gewünschten Drosselwirkung des Ringspalts. In einer weiteren vorteilhaften Ausgestaltung ist die Trennwand zusammen mit dem Hohlfasermembranbündel an ihrem äußeren Umfang fluiddicht in eine Hülse eingebettet, deren Außendurchmesser im wesentlichen mit dem Innendurchmesser des Gehäuses übereinstimmt, wobei aber in die Außenseite der Hülse und/oder in die Innenwand des Gehäuses sich in axialer Richtung des Gehäuses erstreckende Nuten eingebracht sind. Die Hülse ist dann im wesentlichen fest in das Gehäuse eingepresst, so dass die Nuten eine Vielzahl definierter Drosseln darstellen.

Das Hohlfasermembranbündel kann bei derartigen Ausführungsformen auch in seinem gesamten der Trennwand zugewandten Endbereich oder auch über seine gesamte Erstreckung in eine Hülse eingeschoben sein, wobei auch hier die Trennwand fluiddicht mit der Hülse verbunden ist. Im Bereich der Fluidzuläufe oder -abläufe ist dann zur Gewährleistung einer guten Verteilung des Fluids über das Hohlfasermembranbündel die Hülse mit Durchlässen z.B. in Form von Löchern versehen und zweckmäßigerweise so geformt, dass dort ein breiterer Spalt zwischen Gehäuse und Hülse ausgebildet wird als im Bereich der Trennwand. Im Bereich des Dialysatraums liegt die Hülse mit ihrer Außenseite ohne Spalt dicht an der Gehäuseinnenwand an.

Durch eine Ausbildung der Hülse und der Gehäuseinnenwand im Bereich der Trennwand in Form von im Längsschnitt des Membranmoduls gesehen einander mit ihren Keilflächen zugewandten Keilen lässt sich ein Ringspalt ausbilden, dessen Spaltbreite vorzugsweise einstellbar ist. Unterschiedliche Ringspattbreiten können durch Veränderung der Position der Keile zueinander erzeugt werden, wobei der Ringspalt zum einen durch Veränderung der Position des Membranbündels entlang der Längserstreckung des Gehäuses, zum anderen durch einen in der Gehäusewand verschiebbaren Keil eingestellt werden kann.

Auch bei den zuvor beschriebenen Ausführungsformen, bei denen die mindestens eine Drossel in Form von Ringspalten, Ringspaltsegmenten oder Nuten ausgeführt sind, sind die Einlasseinrichtung des Substituatraums und die Auslasseinrichtung des Dialysatraums zweckmäßigerweise an den der Trennwand abgewandten Enden der jeweiligen Räume angeordnet. Bei diesen Ausführungsformen des erfindungsgemäßen Membranmoduls wie auch bei Ausführungsformen, bei denen die Drosseln in Form von Löchern in der Trennwand oder als kurze Kapillaren ausgeführt sind, werden die jeweiligen Membranmodule bei Hemodiafiltrationsverfahren eingesetzt, bei denen eine Nachverdünnung des Bluts mit Substituat erfolgt, d.h. dem Blut wird zunächst im Bereich des Dialysatraums über Ultrafiltration die erforderliche Flüssigkeit entzogen und anschließend im Bereich des Substituatraums Substituat zugeführt. Hierzu strömt das Blut an dem dem Substituatraum abgewandten Ende des Hemodiafilters in die Hohtfasermembranen ein und durchströmt diese in Richtung des dem Substituatraum zugewandten Ende. Das Dialysat durchströmt dann den Dialysatraum entgegengesetzt zur Strömungsrichtung des Bluts.

Bei der Durchführung der Hemodiafiltration ist häufig ein externer Sterilfilter dem eigentlichen Hemodiafilter vorgeschaltet, mittels dessen eine Sterilfiltration der Dialysierfiüssigkeit oder zumindest der als Substituat zugeführten Flüssigkeit durchgeführt wird. In einer vorteilhaften Ausführungsform des erfindungsgemäßen Membranmoduls ist innerhalb des Membranmoduls im Bereich des Substituatraums um das Hohifaserbündel herum ein Sterilfilter angeordnet, der das Hohlfasermembranbündel umschließt. Durch diesen Sterilfilter wird der Substituatraum senkrecht zur Erstrekkung der Hohtfasermembranen in einen äußeren und einen inneren Substituatteilraum unterteilt Mit Vorteil läßt sich als Sterilfilter eine gegebenenfalls plissierte Flachmembran einsetzen. Vorteilhaft ist der Einsatz einer durchgehend mikroporösen Flachmembran. Bevorzugt ist der Sterilfilter dicht gegenüber dem Durchgang von Endotoxinen, d.h. endotoxindicht, und besonders bevorzugt CIS-undurchlässig, um so in der Anwendung die Zuführung eines sterilen endotoxin- und pyrogenfreien und vorzugsweise CIS-freien Substituats zu den Hohtfasermembranen sicherzustellen. Hierbei wird unter einem gegenüber dem Durchtritt von Endotoxinen dichten Sterilfilter ein solcher verstanden, für den bei Filtration einer mit einer Endotoxinkonzentration von bis zu 30 EU/ml kontaminierten Dialysierflüssigkeit mit einer Filtrationsrate von 150 ml/min während einer Dauer von 4 Stunden durch den Sterilfilter hindurch das Filtrat eine Endotoxinkonzentration unterhalb der Nachweisgrenze üblicher Tests, d.h. unterhalb von ca. 0,03 EU/ml aufweist. Die Endotoxinkonzentration wird dabei mittels gängiger LAL-Tests ermittelt, wie sie z.B. von der Fa. BioWhitteker Inc. (Niutti-Test Limulus Amebocyte Lysate Pyrogent®) vertrieben und beschrieben werden.

Der Sterilfilter kann dabei so angeordnet sein, dass in der Anwendung die gesamte dem erfindungsgemäßen Hemodiafilter zugeführte Dialysierflüssigkeit durch den Sterilfilter filtriert wird. In diesem Fall erfolgt im äußeren Substituatteilraum allein eine Verteilung der Dialysierflüssigkeit auf die gesamte Fläche des Sterilfilters, und die mindestens eine Drossel, über die der Substituatraum und der Dialysatraum miteinander in Fluidverbindung stehen, ist im Bereich des inneren Substituatteilraums in die Trennwand integriert.

In der Regel ist jedoch eine Sterilfiltration der gesamten Dialysierflüssigkeit nicht erforderlich, da der letztlich als Dialysat an den Hohlfasermembranen vorbeigeführte Teil der Dialysierflüssigkeit nicht den hohen Reinheitsanforderungen genügen muss, wie dies für das Substituat gilt. Vorzugsweise ist der Sterilfilter daher so im erfindungsgemäßen Membranmodul angeordnet, dass in der Anwendung nur der Teilstrom den Sterilfilter durchströmt, der letztlich als Substituat dem durch die Hohlfasermembranen strömenden Blut zugeführt wird. Dies wird dadurch erreicht, dass die mindestens eine Drossel im Bereich des äußeren Substituateitraums angeordnet ist und nur der äußere Substituatteilraum mit dem Dialysatraum in Fluidverbindung steht. Der innere Substituatteilraum hingegen ist gegenüber dem Dialysatraum fluiddicht abgetrennt. Wie bereits ausgeführt, kann dabei die Drossel z.B. in Form von in die Trennwand integrierten Kapillaren oder in Form eines zwischen der Trennwand und der Gehäuseinnenwand ausgebildeten Ringspalts vorliegen

In Einzelfällen ist es zur Erhöhung des dem Blut zuzuführenden Substituatstroms auch möglich, in die an den Substituatraum angrenzende Vergussmasse, in die die Hohlfasermembranenden eingebettet sind, zusätzliche semipermeable Membranelemente einzubetten, die im dead-end Modus durchströmbar sind und über die der Substituatraum und der auf der anderen Seite der Vergussmasse liegende Raum, der je nach Ausführung des erfindungsgemäßen Membranmoduls der Verteiler- oder der Sammelraum für das Blut ist, in Fluidverbindung stehen. Über diese ebenfalls als Drossel wirkenden Membranelemente kann dem Blut, welches sich dann in dem angrenzenden Sammel- bzw. Verteilerraum befindet, ein weiterer Teil der Dialysierflüssigkeit als Substituat zugeführt werden. Diese semipermeablen Membranelemente können z.B. in Form von an ihrem einen Ende verschlossenen, vorzugsweise durchgehend mikroporösen Kapillarmembranen vorliegen, die in die Vergussmasse eingebettet sind und die mit ihrem verschlossenen Ende in den Substituatraum hineinragen. Diese Membranelemente sind wie der zuvor genannte Sterilfilter bevorzugt dicht gegenüber dem Durchgang von Endotoxinen und besonders bevorzugt CIS-undurchlässig. Hinsichtlich der Definition der Endotoxindichtigkeit bzw. der CIS-Dichtigkeit sowie hinsichtlich der jeweiligen Messmethoden sei auf die zuvor gemachten Ausführungen verwiesen.

In der Anwendung des erfindungsgemäßen Membranmoduls ist eine möglichst gleichmäßige Verteilung von Substituat und Dialysat über den Bündelquerschnitt erforderlich. Eine gleichmäßige Verteilung lässt sich durch eine geeignete Gestaltung des Gehäuses erreichen. Vorzugsweise ist das Gehäuse des erfindungsgemäßen Membranmoduls so ausgebildet, dass es das Bündel der Hohlfasermembranen im überwiegenden Teil des Dialysatraums mit seiner Innenseite eng anliegend umschließt und im Bereich der Trennwand und des Substituatraums sowie gegebenenfalls im Bereich der Vergussmassen eine Erweiterung des Querschnitts aufweist.

Hierdurch sind in einer vorteilhaften Ausgestaltung in diesen Bereichen um das Bündel der Hohtfasermembranen herum ringförmige Räume zum Verteilen der Dialysierflüssigkeit und des Dialysats auf das Hohtfasermembranbündel bzw. zum Sammeln des Dialysats aus dem Membranbündel ausgebildet.

Bevorzugt weist das Bündel zumindest im überwiegenden Teil des Dialysatraums eine auf den Bündelquerschnitt bezogene und über die Erstreckung des Bündels in diesem Bereich im wesentlichen gleichmäßige Packungsdichte der Hohlfasermembranen zwischen 40 und 65 % auf. Es hat sich gezeigt, dass für den erfindungsgemäß eingesetzten Hemodiafilter d.h. den erfindungsgemäßen Membranmodul bei derartigen Packungsdichten eine gute Entfernung der harnpflichtigen Stoffe aus dem Blut ermöglicht wird.

In einer ebenfalls vorteilhaften Ausgestaltung ist das Gehäuse so ausgeformt, dass es das Bündel der Hohtfasermembranen im Bereich des Dialysatraums mit seiner Innenseite eng anliegend umschließt und im Bereich der Vergussmassen sowie der Trennwand und des Substituatraums eine Erweiterung des Gehäusequerschnitts aufweist, und das Hohlfasermembranbündel ist so im Gehäuse angeordnet, dass sich im Bereich der Trennwand und des Substituatraums der Querschnitt des Hohlfasermembranbündels erweitert und damit die Packungsdichte der Hohlfasermembranen in diesem Bereich geringer ist als im überwiegenden Teil des Dialysatraums. Hierdurch sind auch die Hohtfasermembranen im Bündelinneren leicht von der DialysierHüssigkeit bzw. dem Substituat erreichbar und das Substituat strömt gleichmäßig in alle Hohlfasermembranen des Bündels ein. In seinem erweiterten Bereich weist das Bündel vorzugsweise eine auf den jeweiligen Bündelquerschnitt bezogene Pakkungsdichte zwischen 20 und 55 % auf. Besonders bevorzugt ist bei derartigen Ausführungsformen die mindestens eine Drossel in Form von mehreren ringförmig um das Bündel der Hohlfasermembranen herum angeordneten Kapillaren oder als Ringspalt zwischen Trennwand und Gehäuseinnenwand ausgebildet.

Nicht zuletzt aus Gründen der einfachen Herstellbarkeit besteht die Trennwand des erfindungsgemäßen Hemodiafilters bzw. Membranmoduls bevorzugt aus einer Vergussmasse, in die die Hohtfasermembranen so eingebettet sind, dass sie jede Hohlfasermembran umschließt. Besonders bevorzugt bestehen die Trennwand, die erste und die zweite Vergussmasse aus dem gleichen Material. Hierbei können die zur Einbettung von Hohlfasermembranen üblicherweise als Vergussmassen zum Einsatz kommenden Materialien wie Polyurethanharze, Epoxyharze und ähnliche verwendet werden.

Zur Durchführung einer effizienten Hemodiafiltration ist es erforderlich, dass,eine ausreichend hohe Austauschfläche für die Diafiltration zur Verfügung steht, um die harnpflichtigen Stoffe effizient aus dem Blut entfernen zu können. Auf der anderen Seite muss auch eine ausreichende Membranfläche zur Verfügung stehen, um eine sichere Zuführung der erforderlichen Menge an Substituat zum Blut zu ermöglichen. Daher liegt bei dem erfindungsgemäßen Hemodiafilter in Richtung der Erstreckung der Hohtfasermembranen gesehen das Verhältnis L_{d}/ Lₛ der Erstreckung des Dialysatraums L_{d} zur Erstreckung des Substituatraums Lₛ im Bereich zwischen 3 und 20 und besonders bevorzugt zwischen 5 und 15.

Um ebenfalls eine möglichst große Membranfläche für die Substituatzuführung zum Blut und für die Hemodiafiltration zur Verfügung zu haben, sollte die Dicke der Trennwand im erfindungsgemäßen Membranmodul möglichst gering sein. Andererseits ist eine bestimmte Mindestdicke erforderlich, um eine ausreichende Stabilität der Trennwand zu gewährleisten. Daher ist es von Vorteil, wenn die Trennwand eine Dicke zwischen 1 und 15 mm aufweist, und von besonderem Vorteil, wenn sie eine Dicke zwischen 5 und 10 mm aufweist.

Für eine effiziente Hemodiafiltration ist es erforderlich, zur Entfernung insbesondere der langsam diffundierenden hampftichtigen Stoffe mit mittlerem Molekulargewicht einen ausreichend hohen konvektiven Transport zu erzeugen. Zum anderen ist es vor Vorteil wenn für die Zuführung von Substituat zum Blut nur ein relativ kleiner Abschnitt des Hohtfasermembranbündels benötigt wird. Damit ergibt sich, dass über die Membranwand hinweg ein genügend hoher Fittratfluss realisierbar sein muss. Daher weisen die im erfindungsgemäßen Membranmodul enthaltenen Hohtfasermembranen bevorzugt eine Ultrafiltrationsrate für Wasser zwischen 20 und 1500 ml/(h·m²·mmHg) auf, wobei die Uttrafittrationsrate nach der in der DE-A 195 18 624 beschriebenen Methode ermittelt wird, auf deren diesbezügliche Offenbarung sich hier bezogen wird.

Für einen sicheren Betrieb des erfindungsgemäßen Membranmoduls ist wichtig, dass es insbesondere bei der Zuführung des Substituats zum Blut nicht zu einer unerwünschten Verunreinigung des durch die Hohtfasermembranen strömenden Bluts mit Bakterien, Endotoxinen oder Pyrogenen kommt. Entsprechend den obigen Ausführungen kann hierzu die Dialysierflüssigkeit oder zumindest das Substituat in einem separaten oder einem in den erfindungsgemäßen Membranmodul integrierten Sterilfilter einer Sterilfiltration unterzogen werden. Alternativ oder ergänzend kann diese Sterilfiltration jedoch auch in den Hohlfasermembranen des erfindungsgemäßen Membranmoduls selbst erfolgen. In einer bevorzugten Ausführungsform des erfindungsgemäßen Membranmoduls sind daher die Hohlfasermembranen undurchlässig gegenüber Endotoxinen und besonders bevorzugt undurchlässig gegenüber cytokin-induzierenden Substanzen. Hierbei kann die Undurchlässigkeit durch eine entsprechend eingestellte Porengröße der trennaktiven Schicht der Membranen und/oder durch adsorptive Eigenschaften der Hohlfasermembranen erreicht werden. Hinsichtlich der Definition der Endotoxindichtigkeit bzw. der CIS-Dichtigkeit sowie hinsichtlich der jeweiligen Meßmethoden sei auf die weiter oben gemachten Ausführungen verwiesen.

Die erfindungsgemäß eingesetzten Hohlfasermembranen haben vorzugsweise einen Innendurchmesser zwischen 140 und 260 µm, die Wandstärke liegt bevorzugt zwischen 5 und 100 µm und besonders bevorzugt zwischen 20 und 60 µm. Als Membränmaterialien kommen vorzugsweise solche in Frage, die eine gute Blutverträglichkeit aufweisen. Hierzu zählen Polymere aus der Gruppe der cellulosischen Polymeren, wie z.B. Cellulose oder regenerierte Cellulose, modifizierte Cellulose, wie z.B. Celluloseester, Celluloseäther, aminmodifizierte Cellulosen, sowie Mischungen von cellulosischen Polymeren, aus der Gruppe der synthetischen Polymeren wie z.B. Polyacrylnitril und entsprechende Copolymere, Polyaryisulfone und Polyarylethersulfone, wie z.B. Polysulfon oder Polyethersulfon, Polyamide, Polyetherblockamide, Polycarbonate oder Polyester sowie daraus gewonnene Modifikationen, Blends, Mischungen oder Copolymere dieser Polymere. Diesen Polymeren bzw. Polymergemischen können weitere Polymere wie z.B. Polyethylenoxid, Polyhydroxyether, Polyethylenglykol, Polyvinylpyrrolidon, Polyvinylalkohol oder Poiycaprolacton als Zusatzstoffe beigemischt werden. Im Einzelfall kann die Membran auch z.B. einer Oberftächenmodiftkation unterzogen worden sein, um bestimmte Eigenschaften der Membranoberfläche z.B. in Form bestimmter funktioneller Gruppen einzustellen oder um eine Hydrophilierung einer ansonsten hydrophoben Membran an deren Oberflächen zu erreichen, wie dies z.B. in der JP-A 10118472 beschrieben wird

Die Aufmachung des im erfindungsgemäßen Membranmodul angeordneten Bündels von Hohlfasermembranen, d.h. die Anordnung der Hohlfasermembranen zum Bündel kann beliebig sein, wobei eine gute Umströmbarkeit der einzelnen Hohlfasermembranen gewährleistet sein sollte. Bei einer vorteilhaften Aufmachung sind die Hohlfasermembranen zueinander und zur Längsachse des Bündels im wesentlichen parallel und werden über textile Fäden gegeneinander auf Abstand gehalten. Dies kann beispielsweise dadurch erreicht werden, dass vor der Ausbildung zum Bündel die Hohlfasermembranen mittels der textilen Fäden zu einer Matte oder zu einem Bändchen von parallel liegenden Hohtfasermembranen verwoben und anschließend zum Bündel konfiguriert werden. Das im erfindungsgemäßen Membranmodul enthaltene Bündel von Hohlfasermembranen kann auch aus Teilbündeln zusammengesetzt sein, solange jede der Hohtfasermembranen des Bündels in der Anwendung zur Blutbehandlung, zur Filtrierung des Substituats und zur Zuführung des Substituats zum Blut dienen. Ein solcher Aufbau aus Teilbündeln, bei dem die Teilbündel zur Verbesserung der Umströmbarkeit der Hohtfasermembranen mit wcketfäden umwickelt sind und die Hohtfasermembranen innerhalb der Teilbündel über Stützfäden auf Abstand gehalten sind, ist beispielsweise in der EP-A 732 141 beschrieben. Darüber hinaus können die Hohlfasermembranen auch eine Ondulation aufweisen.

Die Erfindung wird nachfolgend anhand der Figuren näher erläutert. Es zeigen in vereinfachter schematischer Darstellung:
- Fig. 1:: einen Längsschnitt durch einen erfindungsgemäßen Membranmodul mit in die Trennwand integrierten Drosseln in Form kurzer Kapillaren, die ringförmig um das Bündel der Hohtfasermembranen herum angeordnet sind,
- Fig. 2:: einen Längsschnitt durch einen erfindungsgemäßen Membranmodul mit in die Trennwand integrierten Drosseln in Form langer Kapillaren, die ringförmig um das Bündel der Hohtfasermembranen herum angeordnet sind,
- Fig. 3:: Segment eines Längsschnitts durch einen erfindungsgemäßen Membranmodul mit einer in eine Hülse eingebetteten Trennwand und mit einem Ringspalt als Drossel, der zwischen Hülse und Gehäuseinnenwand ausgebildet ist,
- Fig. 4:: Segment eines Längsschnitts durch eine weitere Ausführungsform eines erfindungsgemäßen Membranmoduls mit einer in eine Hülse eingebetteten Trennwand und mit einem Ringspalt zwischen Hülse und Gehäuseinnenwand als Drossel,
- Fig. 5:: Segment eines Längsschnitts durch einen erfindungsgemäßen Membranmodul mit einer einstellbaren Drossel in Form eines durch gegeneinander verschiebbare Keile ausgebildeten Ringspalts.
- Fig. 6:: Segment eines Längsschnitts durch einen erfindungsgemäßen Membranmodul mit einem in das Gehäuse integrierten Sterilfilter und Drosseln in Form kurzer Kapillaren im Bereich des inneren Substituatteilraums,
- Fig. 7:: Segment eines Längsschnitts durch einen erfindungsgemäßen Membranmodul mit einem in das Gehäuse integrierten Sterilfilter und Drosseln in Form kurzer Kapillaren im Bereich des äußeren Substituatteilraums,
- Fig. 8:: Segment eines Längsschnitts durch einen erfindungsgemäßen Membranmodul mit einem in das Gehäuse integrierten Sterilfilter und Drosseln in Form eines Ringspalts zwischen Hülse und Gehäuseinnenwand im Bereich des äußeren Substituatteilraums,
- Fig. 9:: Schema eines Hemodiafiltrationssystems, bei dem ein erfindungsgemäßer Membranmodul eingesetzt ist.

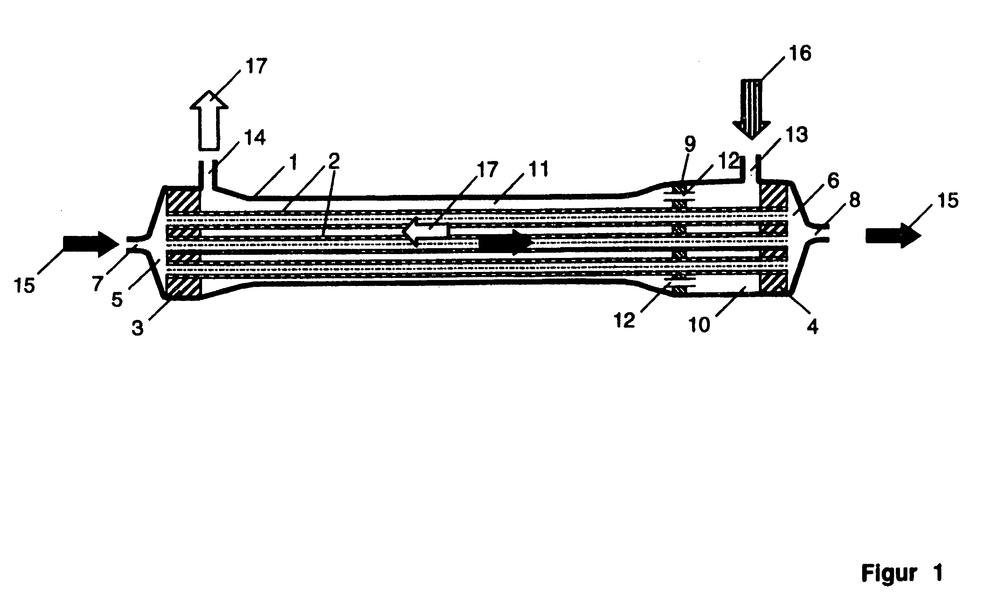

Figur 1 zeigt einen erfindungsgemäßen Membranmodul, welcher bei Hemodiafiltrationsverfahren einsetzbar ist, bei denen eine Nachverdünnung des Bluts mit Substituat erfolgt. Der Membranmodul gemäß Figur 1 weist ein zylinderförmiges Gehäuse 1 auf, in dem ein Bündel von in Richtung der Längserstreckung des Gehäuses orientierten Hohtfasermembranen 2 angeordnet ist. Die Hohlfasermembranen sind mit ihren Enden in Vergussmassen 3,4 fluiddicht eingebettet, welche ihrerseits fluiddicht mit der Innenwand des Gehäuses 1 verbunden sind. Die Hohlfasermembranen sind so in die Vergussmassen 3,4 eingebettet, dass ihre Enden durch die Vergussmassen 3,4 hindurchtreten und ihre Lumina in den Verteilerraum 5 bzw. den Sammelraum 6 münden. Der Verteilerraum 5 weist eine Bluteinlasseinrichtung 7 und der Sammelraum 6 eine Blutauslasseinrichtung 8 auf.

Um die Hohlfasermembranen 2 herum ist zwischen den Vergussmassen 3,4 und der Innenwand des Gehäuses 1 ein Außenraum ausgebildet, der entlang der Erstrekkung der Hohlfasermembranen 2 durch eine quer zu den Hohlfasermembranen 2 verlaufende und im wesentlichen dimensionsstabile Trennwand 9 z.B. aus einer E-poxy- oder Polyurethanvergussmasse in einen Substituatraum 10 und einen Dialysatraum 11 unterteilt ist. Die Trennwand 9 umschließt die einzelnen Hohlfasermembranen 2 und ist für den in Figur 1 dargestellten Fall fluiddicht mit der Gehäuseinnenwand verbunden. In die Trennwand 9 sind als Drosseln kurze Kapillaren 12 eingebettet, welche ringförmig um das Bündel der Hohlfasermembranen 2 herum angeordnet sind. Über diese Kapillaren 12 stehen der Substituatraum 10 und der Dialysatraum 11 miteinander in Fluidverbindung. Um die Kapillaren 12 ringförmig um das Hohlfasermembranbündel anordnen und in der Anwendung eine gute Verteilung der Dialysierflüssigkeit im Substituatraum 10 erreichen zu können, ist das Gehäuse 1 des in Figur 1 gezeigten Membranmoduls im Bereich der Trennwand 9 und im Bereich des Substituatraums 10 in seinem Querschnitt erweitert. Der Substituatraum weist allein eine Einlasseinrichtung 13 für die Dialysierflüssigkeit, der Dialysatraum allein eine Auslasseinrichtung 14 für das Dialysat auf. Im Bereich der Auslasseinrichtung 14 ist der Querschnitt des Gehäuses 1 ebenfalls erweitert, um das Dialysat gleichmäßig aus dem Modul abziehen zu können.

In der Anwendung strömt das Blut, welches durch die Pfeile 15 angedeutet ist, über die Bluteinlasseinrichtung 7 in den Verteilerraum 5, durchströmt die Lumina der Hohtfasermembranen 2, strömt anschließend aus den Hohtfasermembranen 2 in den Sammelraum 6 und wird über die Blutauslasseinrichtung 8 aus dem Membranmodul bzw. Hemodiafilter ausgeleitet. Über die Einlasseinrichtung 13 wird Dialysierflüssigkeit, angedeutet durch den Pfeil 16, in den Substituatraum 10 eingeleitet. Ein Teil der Dialysierflüssigkeit strömt im Bereich des Substituatraums 10 als Substituat in die dort hindurchführenden Hohtfasermembranen 2 ein und vermischt sich mit dem durch die Hohtfasermembranen 2 strömenden Blut. Der größere Teil der Dialysierflüssigkeit strömt über die Drosseln in Form der Kapillaren 12 als Dialysat 17 in den Dialysatraum 11 und durchströmt den Dialysatraum 11 entgegengesetzt zur Strömungsrichtung des Bluts. Hierbei nimmt das Dialysat 17 das über die Wände der Hohlfasermembranen 2 ausströmende Ultrafiltrat zusammen mit den aus dem Blut entfernten harnpflichtigen Substanzen auf. Das mit dem Ultrafiltrat vermischte Dialysat 17 wird über die Auslasseinrichtung 14 aus dem Dialysatraum 11 abgezogen. Bei dem in Figur 1 dargestellten Modul wird dem Blut zunächst das Ultrafiltrat entzogen und anschließend Substituat zugeführt. Es findet also eine Nachverdünnung des Bluts statt.

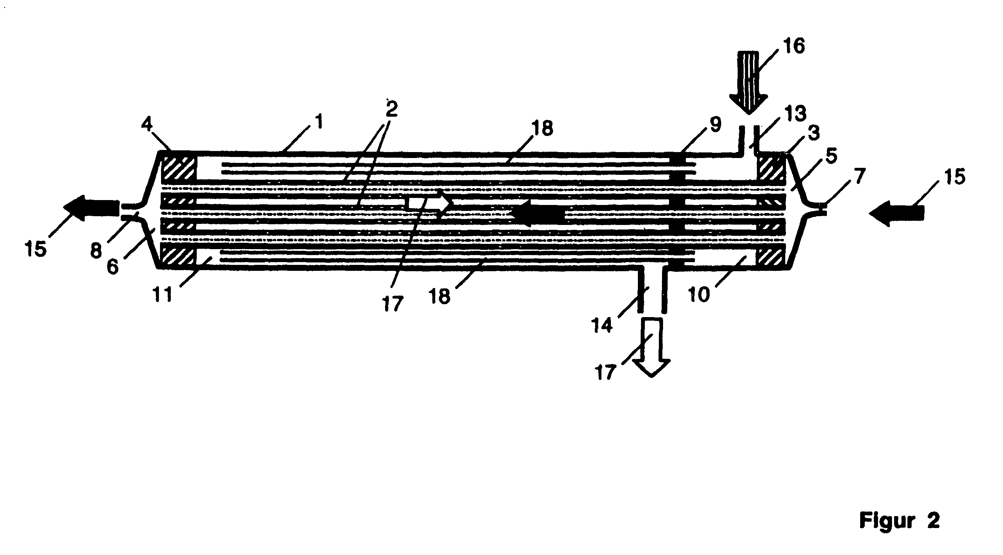

Der in Figur 2 dargestellte erfindungsgemäße Membranmodul ist für Hemodiafiltrationsverfahren geeignet, bei denen eine Vorverdünnung des Bluts erfolgt. In wesentlichen Merkmalen entspricht der Membranmodul gemäß Figur 2 dem in Figur 1 dargestellten Membranmodul, so dass die gleichen Teile mit gleichen Bezugszeichen versehen sind und auf eine erneute ausführliche Beschreibung verzichtet wird. Im Unterschied zu dem Membranmodul der Figur 1 sind in der Trennwand 9 des Membranmoduls der Figur 2 jedoch lange Kapillaren 18 als Drosseln ringförmig um das im Gehäuse 1 dieses Moduls befindliche Bündel von Hohtfasermembranen 2 eingebettet. Diese Kapillaren 18 erstrecken sich durch den Dialysatraum 11 hindurch bis kurz vor die der Blutauslasseinrichtung 8 zugewandten Vergussmasse 4. Derartige lange Kapillaren 18 können vorteilhaft zueinander parallel in einer Matte eingebunden werden, die sich dann auf einfache Weise um das Hohlfasermembranbündel herumwickeln läßt. Danach können dann die Kapillaren zusammen mit den Hohlfasermembranen in die Trennwand eingebettet werden.

Bei der Hemodiafiltration wird das Blut über die Bluteinlasseinrichtung 7 und den Verteilerraum 5 in die Hohlfasermembranen 2 geleitet und durchströmt diese. Hierbei wird dem Blut im Bereich des Substituatraums 10 Substituat zugeführt und das Blut dabei mit Substituat verdünnt, bevor es auf seinem weiteren Weg durch die Hohlfasermembranen 2 den Bereich des Dialysatraums 11 durchläuft, in dem ihm über Ultrafittration durch die Wände der Hohtfasermembranen die erforderliche Flüssigkeitsmenge entzogen wird und dabei die harnpflichtigen Stoffe entfernt werden. Das gereinigte und auf den erforderlichen Flüssigkeitsgehalt eingestellte Blut verlässt den erfindungsgemäßen Membranmodul über die Blutauslasseinrichtung 8.

Die Dialysierflüssigkeit 16 wird über die Einlasseinrichtung 13 in den Substituatraum 10 eingeleitet, der bei der Modulausführung gemäß Figur 2 an dem der Bluteinlasseinrichtung 7 zugewandten Ende der Hohtfasermembranen 2 angeordnet ist. Im Substituatraum 10 strömt ein Teil der Dialysierflüssigkeit als Substituat durch die Wände der Hohtfasermembranen 2 in das durch die Hohlfasermembranen strömende Blut und verdünnt dieses. Der größere Teil der Dialysierflüssigkeit strömt als Dialysat 17 über die in der Trennwand 9 als Drosseln eingebetteten langen Kapillaren 18 in den Dialysatraum. Das Dialysat 17 veriässt die Kapillaren in der Nähe der der Blutauslasseinrichtung 8 zugewandten Vergussmasse 4 und durchströmt den Dialysatraum entgegengesetzt zur Strömungsrichtung des Bluts in Richtung auf die Trennwand 9. Hierbei nimmt es das Ultrafiltrat mit den aus dem Blut entfernten hampflichtigen Substanzen auf und wird über die in der Nähe der Trennwand 9 liegende Auslasseinrichtung 14 aus dem Dialysatraum 11 abgezogen.

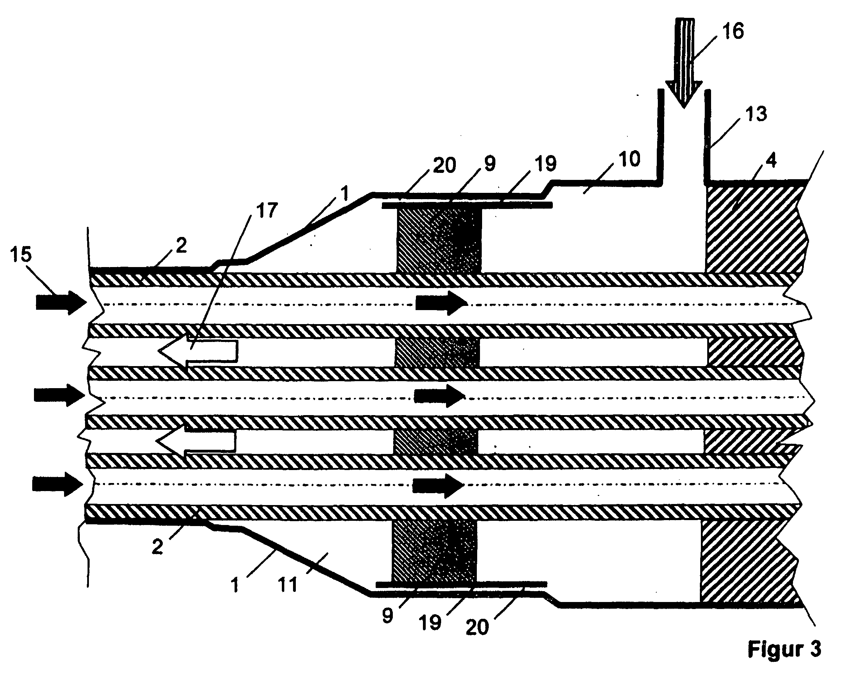

Figur 3 zeigt in gegenüber den Figuren 1 und 2 vergrößerter Darstellung ein den Substituatraum 10 und die Trennwand 9 umfassendes Segment eines erfindungsgemäßen Membranmoduls. Auch die in Figur 3 und den folgenden Figuren gezeigten Membranmodule bzw. Segmente von Membranmodulen entsprechen in wesentlichen Teilen dem in Figur 1 dargestellten Membranmodul, so dass die gleichen Teile mit gleichen Bezugszeichen versehen sind und auf eine erneute ausführliche Beschreibung verzichtet wird. In Figur 3 ist eine Ausführungsform dargestellt, bei der die Trennwand 9 zusammen mit dem Bündel der Hohlfasermembranen 2 fluiddicht in eine formstabile Hülse 19 eingebettet ist. Die Hülse 19 weist gegenüber der Innenwand des Gehäuses einen Abstand auf, wodurch ein die Drossel darstellender Ringspalt 20 ausgebildet ist. Die Drosselwirkung des Ringspalts kann auf einfache Weise über dessen Spaltweite und -länge durch Auswahl der Hülse definiert voreingestellt werden. Zur Stabilisierung des Spalts 20 können zwischen Hülse 19 und Innenwand des Gehäuses 1 Abstandhalter eingebracht werden. Bei dem in Figur 3 dargestellten Membranmodul erfolgt in der Hemodiafiltration eine Nachverdünnung des Bluts mit Substituat. Das Blut 15 durchläuft die Hohlfasermembranen 2 zunächst im Bereich des Dialysatraums 11 und anschließend im Bereich des Substituatraums 10.

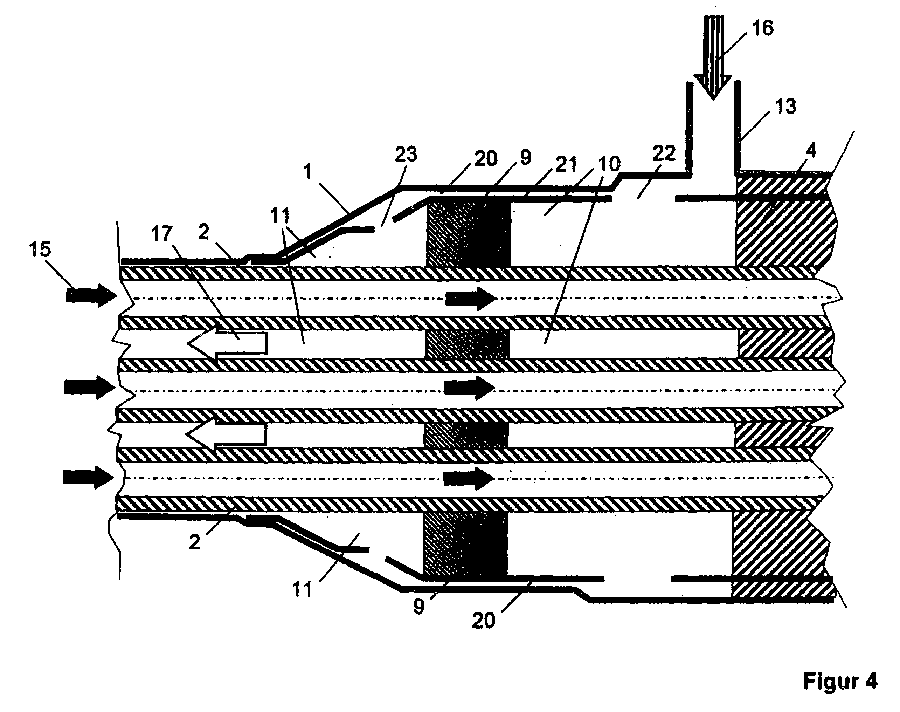

Bei dem in Figur 4 dargestellten Segment eines erfindungsgemäßen Membranmoduls wird ebenfalls ein die Drossel ausbildender Ringspalt 20 zwischen einer formstabilen Hülse 21 und der Innenwand des Gehäuses 1 ausgebildet. Die Hülse, die zur Vereinfachung der Fertigung des erfindungsgemäßen Membranmoduls im vorliegenden Fall das gesamte dem Substituatraum 10 zugewandte Ende des Hohlfasermembranbündels umschließt, ist an ihrem einen Ende fest in das Gehäuse eingeschoben und an ihrem anderen Ende zusammen mit den Hohlfasermembranen in die Vergussmasse 4 eingebettet. Hierdurch wird gleichzeitig eine stabile Positionierung und Ausbildung des Spalts 20 erreicht. Durch eine geeignete Formgebung von Hülse 21 und Gehäuse 1 im Zulaufbereich des Substituatraums 10 und im Zulaufbereich des Dialysatraums 11 sowie durch Öffnungen 22,23 in der Hülse wird eine homogene Verteilung des Substituats bzw. des Dialysats 17 auf die Hohlfasermembranen erreicht. Die Dialysierflüssigkeit 16 wird über die Einlasseinrichtung 13 dem Membranmodul zugeführt und verteilt sich im Bereich des erweiterten Abstands zwischen Hülse 21 und Gehäuse 1 gleichmäßig über den Umfang des Moduls. Durch Öffnungen 22 in der Hülse 21 strömt das Substituat in den Substituatraum 10 ein. Das Dialysat 17 strömt über den die Drossel ausbildenden Spalt 20 in einen Bereich mit erweiterter Spaltweite zwischen Hülse 21 und Innenwand des Gehäuses 1, wo eine Vergleichmäßigung über dem Umfang erfolgt, bevor das Dialysat 17 durch Öffnungen 23 in den Dialysatraum 11 einströmt.

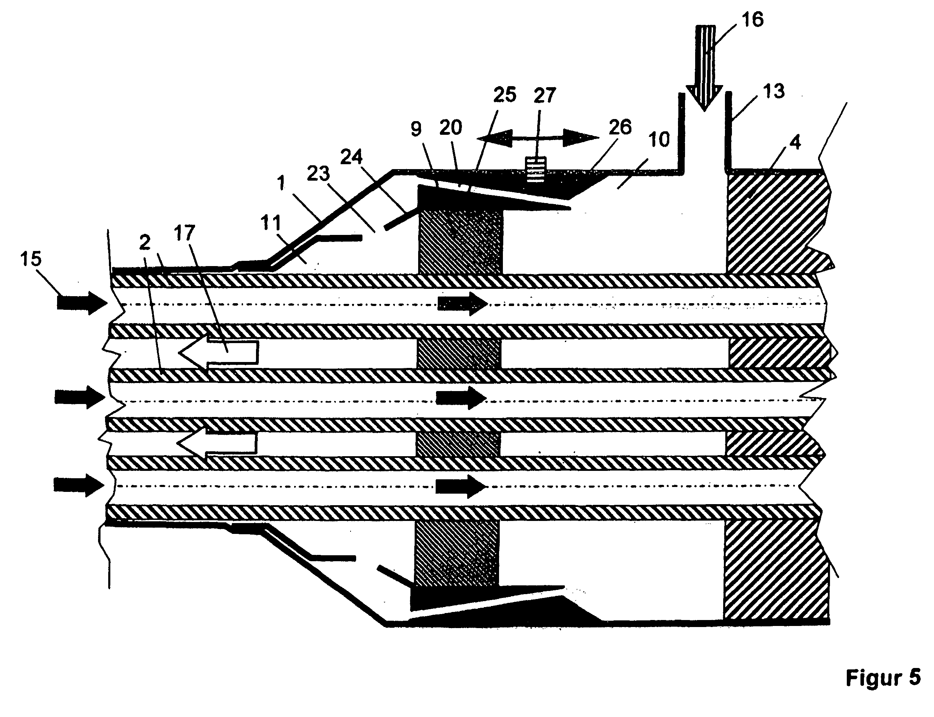

Figur 5 zeigt eine weitere Variante eines erfindungsgemäßen Membranmoduls, bei dem das Membranbündel zusammen mit der Trennwand 9 in eine Hülse eingeschoben ist. Die Hülse 24 ist im Bereich der Trennwand 9 in Form eines Keils 25 ausgeführt, wobei die Keilfläche nach außen zeigt. An die innenwand des Gehäuses 1 ist in diesem Bereich ebenfalls ein Keil 26 angebracht, dessen Keilfläche nach innen zeigt und der den Keil 25 der Hülse 24 ringförmig umschließt. Zwischen den Keilflächen der Keile 25,26 ist ein Ringspalt 20 ausgebildet, der als Drossel fungiert. Die Spaltweite des Ringspalts und damit die Drosselwirkung ist bei der in Figur 5 gezeigten Ausführung durch Verschieben des an der Gehäusewand angebrachten Keils 26 einstellbar. Wie angedeutet kann der Keil 26 über ein durch die Gehäusewand hindurchtretendes Verstellglied 27 in Längserstreckung des Gehäuses von außen verstellt werden.

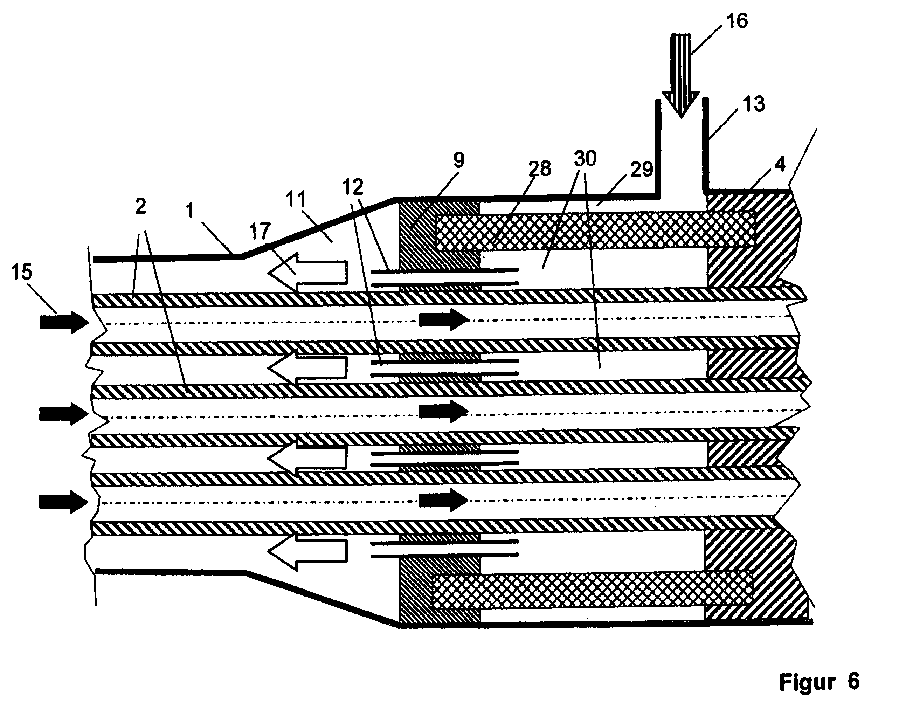

In Figur 6 ist ein Segment eines erfindungsgemäßen Membranmoduls mit einem im Gehäuse 1 integrierten Sterilfilter 28 zur Sterilfiltration der Dialysierflüssigkeit 16 dargestellt. Der Sterilfilter 28, vorzugsweise in Gestalt einer bakterien- und endotoxindichten Flachmembran, umschließt das Hohlfasermembranbündel im Bereich des Substituatraums und teilt den Substituatraum in einen äußeren Substituatteilraum 29, der über die Trennwand 9 vom Dialysatraum 11 fluiddicht getrennt ist, und in einen inneren Substituatteilraum 30, der über als Drosseln fungierende kurze Kapillaren 12 mit dem Dialysatraum 11 in Fluidverbindung steht. Der Sterilfilter lässt sich auf einfache Weise zusammen mit den Hohlfasermembranen 2 in die Vergussmasse 4 und die Trennwand 9 fluiddicht einbetten. Bei der in Figur 6 gezeigten Ausführungsform des erfindungsgemäßen Hemodiafilters wird in der Anwendung die über die Einlasseinrichtung 13 in das Gehäuse eingeleitete Dialysierflüssigkeit im äußeren Substituatteilraum über den gesamten Umfang gleichmäßig verteilt und strömt vollständig durch den Sterilfilter 28. Durch die in die Trennwand 9 im Bereich des inneren Substituatteilraums 30 über den gesamten Bündelquerschnitt verteilten kurzen Kapillaren 12 wird das Dialysat 17 in den Dialysatraum 11 geleitet.

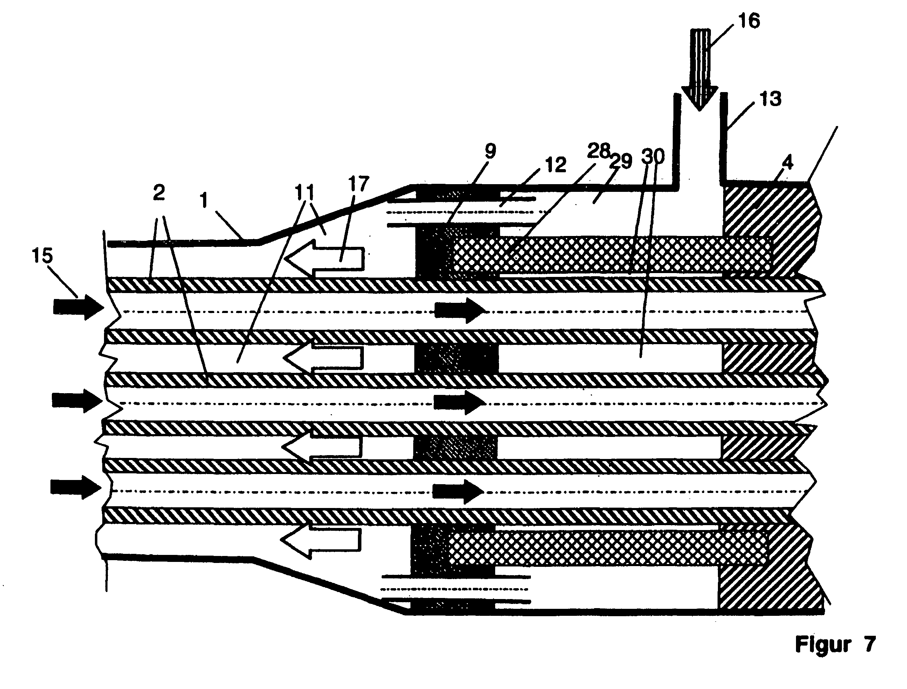

Figur 7 zeigt ebenfalls einen erfindungsgemäßen Membranmodul mit integriertem Sterilfilter 28, der den Substituatraum in einen äußeren Substituatteilraum 29 und einen inneren Substituatteilraum 30 unterteilt. Im Unterschied zu dem Modul gemäß Figur 6 sind bei dem Modul gemäß Figur 7 die als Drosseln fungierenden kurzen Kapillaren 12 so ringförmig um das Hohlfasermembranbündel in der Trennwand angeordnet, dass nur der äußere Substituatteilraum 29 mit dem Dialysatraum 11 in Fluidverbindung steht. Der innere Substituatteilraum 30 hingegen ist fluiddicht gegenüber dem Dialysatraum 11 abgetrennt. Bei dieser Ausführungsform wird nur der Teil der Dialysierflüssigkeit 16, der über die Wände der Hohtfasermembranen 2 dem Blut als Substituat zugeführt wird, der Sterilfiltration unterzogen. Der überwiegende Teil der Dialysierflüssigkeit 16 strömt als Dialysat 17, welches nicht den hohen Reinheitsanforderungen entsprechen muss wie das Substituat, ohne den Sterilfilter 28 zu durchlaufen aus dem äußeren Substituatteilraum 29 über die Kapillaren 12 in der Dialysatraum 11.

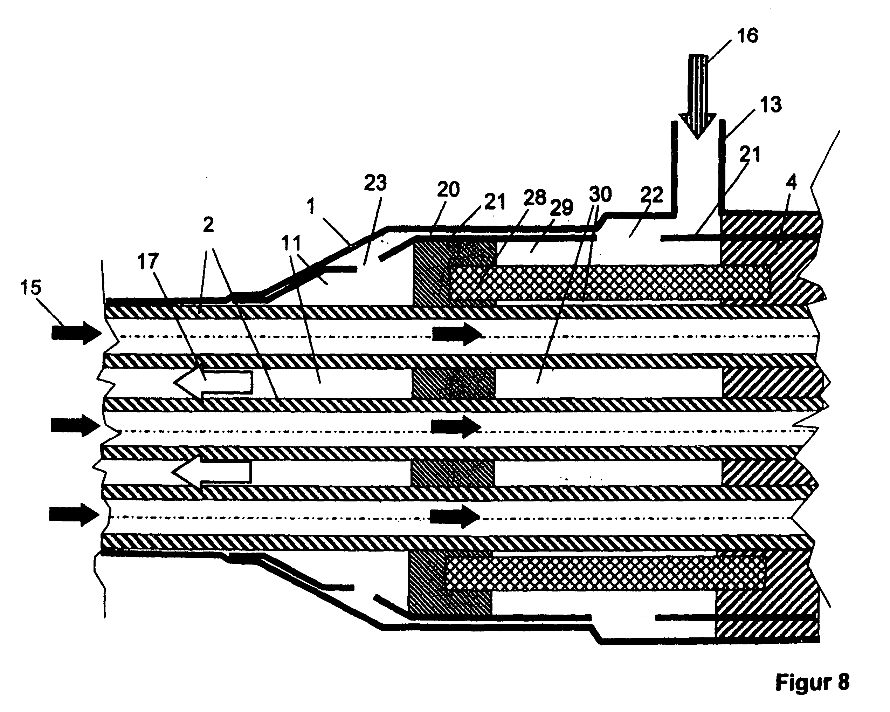

Der in Figur 8 dargestellte erfindungsgemäße Membranmodul ist vom Aufbau her ähnlich dem der Figur 7 und weist einen in den Membranmodul integrierten Sterilfilter 28 auf, der den Substituatraum in einen äußeren Substituatteilraum 29 und einen inneren Substituatteilraum 30 unterteilt, wobei der äußere Substituatteilraum 29 mit dem Dialysatraum 11 in Fluidverbindung steht. Hierdurch wird beim Einsatz dieses Membranmoduls allein der letztlich als Substituat dem Blut zuzuführende Teil der Dialysierflüssigkeit 16 über den Sterilfilter 28 gefiltert, wohingegen der Dialysatteil 17 ohne diese Sterilfiltrierung in den Dialysatraum 11 übergeleitet wird. Die Drossel ist wie auch in dem in Figur 4 gezeigten Modul durch einen Ringspalt 20 gebildet, der im Bereich der Trennwand 9 zwischen einer Hülse 21 und der Innenwand des Gehäuses 1 gebildet ist. Die Hülse 21 der in Figur 8 gezeigten Ausführungsform entspricht derjenigen, die auch bei dem Membranmodul der Figur 4 eingesetzt ist, so dass hinsichtlich der Merkmale der Hülse auf die Ausführungen zu Figur 4 verwiesen wird.

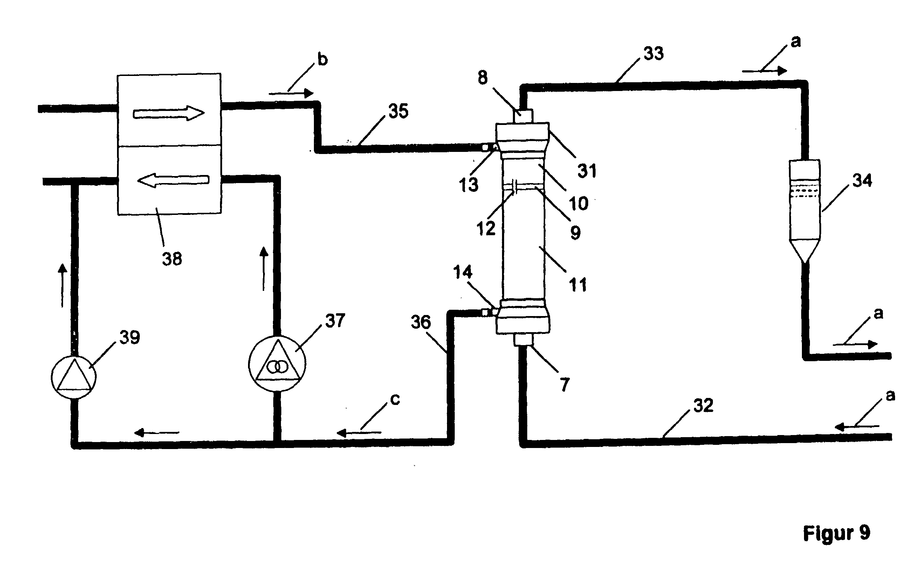

In Figur 9 ist vereinfacht der grundsätzliche Aufbau eines Hemodiafiltrationssystems dargestellt, in welchem ein erfindungsgemäßer Membranmodul eingesetzt ist. Bei dem in Figur 9 dargestellten, den erfindungsgemäßen Membranmodul bzw. Hemodiafilter 31 enthaltenden Hemodiafiltrationssystem wird das dem Patienten entnommene Blut in Pfeilrichtung a über eine Blutzuführleitung 32 der Bluteinlasseinrichtung 7 des Hemodiafilters 31 zugeführt und durch das Lumen der im Membranmodul angeordneten Hohtfasermembranen geleitet. Das gereinigte Blut wird über die Blutauslasseinrichtung 8 des Hemodiafilters 31 aus diesem abgeleitet, in Pfeilrichtung a über die Blutabführleitung 33 in eine Tropfkammer 34 geleitet und von dort dem Patienten wieder zugeführt.

Die Dialysierflüssigkeit wird in Pfeilrichtung b über die Dialysierflüssigkeitsleitung 35 und die Einlasseinrichtung 13 für die Dialysierflüssigkeit in den Substituatraum 10 des Hemodiafilters 31 geleitet. Zur Druckerhöhung kann in die Dialysierflüssigkeitsleitung 35 eine Zentrifugalpumpe eingebaut sein. Aus dem Substituatraum 10 strömt ein Teil der Dialysierflüssigkeit als Substituat über die Wände der im Hemodiafilter 31 angeordneten Hohlfasermembranen in das Lumen der Hohlfasermembranen, wo das Substituat mit dem das Lumen durchströmenden Blut vermischt wird. Der überwiegende Teil der Dialysierflüssigkeit strömt über die in der Trennwand 9 angeordneten Drosseln, die in dieser Darstellung als kurze Kapillaren 12 angedeutet sind, als Dialysat in den Dialysatraum 11. Das im Dialysatraum 11 mit dem aus dem Blut entzogenen Ultrafiltrat angereicherte Dialysat verlässt über die Auslasseinrichtung 14 für das Dialysat den Modul und wird über die Dialysatleitung 36 in Pfeilrichtung c mittels der Dialysatflusspumpe 37 abgezogen. Über die Bitanziereinheit 38 erfolgt eine Kontrolle des Dialysierflüssigkeits-/Dialysatkreislaufs und über die Ultrafiltratpumpe 39 eine Einstellung des im Bereich des Dialysatraums 11 dem Blut entzogenen Nettofiltratstroms. Die Bilanziereinheit 38 arbeitet dabei so, dass der über die Pumpe 37 geförderte Volumenstrom durch einen gleich großen Volumenstrom an frischer Dialysierflüssigkeit ersetzt wird.

Das Hemodiafiltrationssystem gemäß Figur 9 ist aufgrund der Verwendung des erfindungsgemäßen Membranmoduls gegenüber bekannten Hemodiafiltrationssystemen deutlich vereinfacht. So sind beispielsweise bei on-line Hemodiafiltrationssystemen, wie sie z.B. aus der DE-A 196 07 162 bekannt sind, stets separate Vorrich tungen erforderlich, um einen Teil der Dialysierflüssigkeit als Substituat aus der Zuführungsleitung für die Dialysierflüssigkeit zu entnehmen und über eine separate Zuführung dem Blutkreislauf zuzuführen, wobei jedoch über eine zentrale Bilanziereinheit der Flüssigkeitshaushalt des Patienten unter Beachtung aller zugesetzten und entzogenen Flüssigkeiten zentral gesteuert wird, d.h. auch das Substituat wird durch die Bilanziereinheit direkt erfasst. Bei Verwendung des erfindungsgemäßen Membranmoduls sind im Hemodiafittrationssystem keine separaten Vorrichtungen wie Leitungen oder Regelorgane zur Zuführung des Substituats zum Blutkreislauf erforderlich, da die Aufteilung der Dialysierflüssigkeit in Dialysat und Substituat im Membranmodul selbst über die erfindungsgemäß eingesetzte mindestens eine Drossel erfolgt.

## Patentansprüche

1. Membranmodul zur Hemodiafiltration, umfassend ein zylinderförmiges Gehäuse (1) mit einer Längserstreckung, in welchem ein in Richtung der Längserstrekkung des Gehäuses (1) orientiertes Bündel von lumenseitig durchströmbaren Hohlfasermembranen (2) mit semipermeabler Wand angeordnet ist, deren Enden in eine erste und eine zweite mit der Gehäuseinnenwand fluiddicht verbundene Vergussmasse (3,4) so fluiddicht eingebettet sind, dass um die Hohlfasermembranen (2) herum ein von der ersten und der zweiten Vergussmasse (3,4) sowie der Gehäuseinnenwand begrenzter Außenraum ausgebildet wird, der entlang der Längserstreckung des Gehäuses (1) durch eine jede Hohlfasermembran (2) umschließende und zu den Hohtfasermembranen (2) im wesentlichen quer verlaufende Trennwand (9) aus einem im wesentlichen dimensionsstabilen Material in einen Dialysatraum (11) und einen Substituatraum (10) unterteilt wird, wobei der Dialysatraum (11) und der Substituatraum (10) jeweils zumindest eine Öffnung zur Einleitung bzw. zur Ausleitung eines Fluids aufweisen (13,14), **dadurch gekennzeichnet, dass** im Außenraum mindestens eine Drossel angeordnet ist, über die der Dialysatraum (11) und der Substituatraum (10) miteinander in Fluidverbindung stehen.

2. Membranmodul nach Anspruch 1, **dadurch gekennzeichnet, dass** die Trennwand (9) fluiddicht mit der Gehäuseinnenwand verbunden und die mindestens eine Drossel in die Trennwand (9) integriert ist.

3. Membranmodul nach Anspruch 2, **dadurch gekennzeichnet, dass** die in die Trennwand (9) integrierte mindestens eine Drossel mindestens eine Kapillare (12,18) ist, über deren Lumen der Dialysatraum (11) und der Substituatraum (10) miteinander in Fluidverbindung stehen.

4. Membranmodul nach Anspruch 3, **dadurch gekennzeichnet, dass** sich die mindestens eine Kapillare (18) durch den Dialysatraum hindurch erstreckt und im Bereich vor der den Dialysatraum (11) begrenzenden Vergussmasse (4) endet und dass die Öffnung des Dialysatraums (11) zur Trennwand (9) benachbart ist.

5. Membranmodul nach einem oder mehreren der Ansprüche 3 oder 4, **dadurch gekennzeichnet, dass** in die Trennwand (9) mehrere Kapillaren (12,18) eingebracht sind, die um das Bündel der Hohlfasermembranen (2) herum angeordnet sind.

6. Membranmodul nach Anspruch 3, **dadurch gekennzeichnet, dass** in die Trennwand (9) eine einzelne, sich durch den Dialysatraum (11) hindurch erstrekkende Kapillare (12,18) eingebracht ist, die zentral im Bündel der Hohlfasermembranen (2) angeordnet ist.

7. Membranmodul nach Anspruch 1, **dadurch gekennzeichnet, dass** die mindestens eine Drossel ein Ringspalt (20) ist, der zwischen der Trennwand (9) und der Gehäuseinnenwand ausgebildet ist.

8. Membranmodul nach Anspruch 1, **dadurch gekennzeichnet, dass** die Trennwand (9) in eine Hülse (19,21,24) eingebettet ist und die mindestens eine Drossel ein Ringspalt (20) ist, der zwischen der Außenseite der Hülse (19,21,24) und der Gehäuseinnenwand ausgebildet ist.

9. Membranmodul nach Anspruch 1, **dadurch gekennzeichnet, dass** mehrere Drosseln in Form von Ringspaltsegmenten in der Trennwand (9) um das Bündel der Hohtfasermembranen (2) herum oder an der Trennwand (9) an deren äußerem Umfang angeordnet sind.

10. Membranmodul nach Anspruch 1, **dadurch gekennzeichnet, dass** die mindestens eine Drossel einstellbar ist.

11. Membranmodul nach einem oder mehreren der Ansprüche 1 bis 10, **dadurch gekennzeichnet, dass** in Richtung der Längserstreckung des Gehäuses (1) gesehen das Verhältnis L_{d}/ Lₛ der Erstreckung L_{d} des Dialysatraums (11) zur Erstreckung Lₛ des Substituatraums (10) im Bereich zwischen 3 und 20 liegt.

12. Membranmodul nach Anspruch 11, **dadurch gekennzeichnet, dass** das Verhältnis zwischen 5 und 15 liegt.

13. Membranmodul nach einem oder mehreren der Ansprüche 1 bis 12, **dadurch gekennzeichnet, dass** die Trennwand (9) aus einer Vergussmasse besteht.

14. Membranmodul nach Anspruch 13, **dadurch gekennzeichnet, dass** die Trennwand (9), die erste und die zweite Vergussmasse (3,4) aus dem gleichen Material bestehen.

15. Membranmodul nach einem oder mehreren der Ansprüche 1 bis 14, **dadurch gekennzeichnet, dass** die Trennwand (9) eine Dicke zwischen 1 und 15 mm aufweist.

16. Membranmodul nach einem oder mehreren der Ansprüche 1 bis 15, **dadurch gekennzeichnet, dass** das Gehäuse (1) im Bereich des Dialysatraums (11) das Bündel der Hohlfasermembranen (2) mit seiner Innenseite eng anliegend umschließt und im Bereich der Trennwand (9) und des Substituatraums (10) eine Erweiterung des Querschnitts aufweist.

17. Membranmodul nach einem oder mehreren der Ansprüche 1 bis 16, **dadurch gekennzeichnet, dass** im Bereich des Substituatraums (10) um das Bündel der Hohlfasermembranen (2) herum ein das Bündel umschließender Sterilfilter (28) angeordnet ist.

18. Membranmodul nach Anspruch 17, **dadurch gekennzeichnet, dass** der Sterilfilter (28) eine mikroporöse Flachmembran ist.

19. Membranmodul nach einem oder mehreren der Ansprüche 1 bis 18, **dadurch gekennzeichnet, dass** die Hohlfasermembranen (2) eine Ultrafiltrationsrate für Wasser zwischen 20 und 1500 ml/(h·m²·mmHg) aufweisen.

20. Membranmodul nach einem oder mehreren der Ansprüche 1 bis 19, **dadurch gekennzeichnet, dass** die Hohlfasermembranen (2) endotoxindicht sind.

## Claims

1. Membrane module for hemodiafiltration, comprising a cylinder-shaped housing (1) with a longitudinal extent, in which housing a bundle of hollow-fiber : membranes (2) with semipermeable walls and capable of supporting fluid flow through their lumina is arranged in the direction of the longitudinal extent of the housing (1), the ends of the hollow-fiber membranes being embedded in a fluid-tight manner in first and second sealing compounds (3,4) joined to the housing inner wall in a fluid-tight manner, such that an exterior space delimited by the first and second sealing compounds (3,4) and the housing inner wall is formed around the hollow-fiber membranes (2), the exterior space along the longitudinal extent of the housing (1) being divided into a dialyzate space (11) and a substituate space (10) by a dividing wall (9) that is made from a substantially dimensionally stable material, encloses each hollow-fiber membrane (2), and is arranged substantially transversely to the hollow-fiber membranes (2), the dialyzate space (11) and substituate space (10) each having at least one opening (13, 14) for introducing or draining a fluid, **characterized in that** at least one throttle is arranged in the exterior space via which the dialyzate space (11) and substituate space (10) are in fluid communication with each other.

2. Membrane module according to Claim 1, **characterized in that** the dividing wall (9) is joined to the housing inner wall in a fluid-tight manner and the at least one throttle is integrated into the dividing wall (9).

3. Membrane module according to Claim 2, **characterized in that** the at least one throttle integrated into the dividing wall (9) is at least one capillary (12,18), via the lumen of which the dialyzate space (11) and substituate space (10) are in fluid communication with each other.

4. Membrane module according to Claim 3, **characterized in that** the at least one capillary (18) extends through the dialyzate space and terminates in the area of sealing compound (4) delimiting the dialyzate space (11) and that the opening of the dialyzate space (11) is adjacent to the dividing wall (9).

5. Membrane module according to one or more of Claims 3 or 4, **characterized in that** a plurality of capillaries (12,18) are inserted into the dividing wall (9), the capillaries being arranged around the bundle of hollow-fiber membranes (2).

6. Membrane module according to Claim 3, **characterized in that** a single capillary (12,18) extending through the dialyzate space (11) is inserted into the dividing wall (9), the capillary being centrally arranged in the bundle of hollow-fiber membranes (2).

7. Membrane module according to Claim 1, **characterized in that** the at least one throttle is an annular gap (20) that is formed between the dividing wall (9) and the housing inner wall.

8. Membrane module according to Claim 1, **characterized in that** the dividing wall (9) is embedded in a sleeve (19,21,24) and that the at least one throttle is an annular gap (20) that is formed between the outside of the sleeve (19,21,24) and the housing inner wall.

9. Membrane module according to Claim 1, **characterized in that** a plurality of throttles are arranged in the form of annular-gap segments in the dividing wall (9) around the bundle of hollow-fiber membranes (2) or along the periphery of the dividing wall (9).

10. Membrane module according to Claim 1, **characterized in that** the at least one throttle is adjustable.

11. Membrane module according to one or more of Claims 1 to 10, **characterized in that**, viewed in the direction of the longitudinal extent of the housing (1), the ratio L_{d}/Lₛ of the length L_{d} of the dialyzate space (11) to the length Lₛ of the substituate space (10) is between 3 and 20.

12. Membrane module according to Claim 11, **characterized in that** the ratio is between 5 and 15.

13. Membrane module according to one or more of Claims 1 to 12, **characterized in that** the dividing wall (9) consists of a sealing compound.

14. Membrane module according to Claim 13, **characterized in that** the dividing wall (9) and first and second sealing compounds (3, 4) are made of the same material.

15. Membrane module according to one or more of Claims 1 to 14, **characterized in that** the dividing wall (9) has a thickness between 1 and 15 mm.

16. Membrane module according to one or more of Claims 1 to 15, **characterized in that** the housing (1) in the area of dialyzate space (11) tightly encloses the bundle of hollow-fiber membranes (2) with its inside and exhibits an expanded cross-section in the area of dividing wall (9) and substituate space (10).

17. Membrane module according to one or more of Claims 1 to 16, **characterized in that**, in the area of substituate space (10), a sterile filter (28) is arranged around the bundle of hollow-fiber membranes (2) and encloses the bundle.

18. Membrane module according to Claim 17, **characterized in that** the sterile filter (28) is a microporous flat membrane.

19. Membrane module according to one or more of claims 1 to 18, **characterized in that** the hollow-fiber membranes (2) have an ultrafiltration rate for water between 20 and 1500 ml/(h · m² · mmHg).

20. Membrane module according to one or more of Claims 1 to 19, **characterized in that** the hollow-fiber membranes (2) are impermeable to endotoxins.

## Revendications

1. Module à membrane pour hémodiafiltration comprenant un boîtier en forme de cylindre (1) allongé dans le sens longitudinal, dans lequel est disposé un faisceau de membranes en fibres creuses (2) à paroi semi-perméable, à travers lesquelles peut passer un fluide, orientées dans la direction de la longueur du boîtier (1), les extrémités desdites membranes étant noyées dans une première et dans une deuxième masses de scellement (3, 4), reliées, de manière étanche au fluide, à la paroi du boîtier, de telle sorte qu'une chambre externe, limitée par la première et la deuxième masses de scellement (2, 4) ainsi que par la paroi du boîtier, soit formée autour des membranes en fibres creuses (2), laquelle chambre externe est divisée, sur l'étendue longitudinale du boîtier (1), en une chambre à dialyse (11) et une chambre à substituant (10) par une paroi de séparation en matériau de dimensions sensiblement stables, qui, entourant chaque membrane en fibres creuses (2), s'étend sensiblement perpendiculairement par rapport aux dites membranes en fibres creuses (2), la chambre à dialyse (11) et la chambre à substituant (10) présentant chacune au moins une ouverture (13, 14) pour l'introduction et l'évacuation d'un fluide, **caractérisé en ce que**, dans la chambre externe, est disposée au moins un étranglement par l'intermédiaire duquel la chambre à dialyse (11) et la chambre à substituant (10) sont mises en communication pour le fluide.

2. Module à membrane selon la revendication 1, **caractérisé en ce que** la paroi de séparation (9) est reliée de manière étanche au fluide à la paroi intérieure du boîtier et qu'un étranglement au moins est intégré dans ladite paroi de séparation (9).

3. Module à membrane selon la revendication 2, **caractérisé en ce que** l'étranglement, au moins prévu, intégré dans la paroi de séparation (9), est un capillaire (12, 18) par l'intermédiaire du lumen duquel la chambre à dialyse (11) et la chambre à substituant (10) sont mises en communication pour le fluide.

4. Module à membrane selon la revendication 3, **caractérisé en ce que** le capillaire, au moins prévu, (18), s'étend à travers la chambre à dialyse et se termine dans le secteur situé en amont de la masse de scellement (4) qui limite ladite chambre à dialyse (11), et que l'ouverture de la chambre à dialyse (11) avoisine la paroi de séparation (9).

5. Module à membrane selon l'une des revendications 3 ou 4, **caractérisé en ce que** plusieurs capillaires (12, 18) sont prévus dans la paroi de séparation (9), lesquels sont disposés tout autour du faisceau de membranes en fibres creuses (2).

6. Module à membrane selon la revendication 3, **caractérisé en ce que**, dans la paroi de séparation (9), est prévu un capillaire individuel (12, 18) qui, s'étendant à travers la chambre à dialyse (11), est disposé au centre du faisceau de membranes en fibres creuses (2).

7. Module à membrane selon la revendication 1, **caractérisé en ce que** l'étranglement, qui est au moins prévu, est une rainure annulaire (20) qui est formée entre la paroi de séparation (9) et la paroi intérieure du boîtier.

8. Module à membrane selon la revendication 1, **caractérisé en ce que** la paroi de séparation (9) est noyée dans un manchon (19, 21, 24) et que l'étranglement, au moins prévu, est une rainure annulaire (20) qui est formée entre la surface extérieure du manchon (19, 21, 24) et la paroi intérieure du boîtier.

9. Module à membrane selon la revendication 1, **caractérisé en ce que** plusieurs étranglements en forme de segments de rainure annulaire sont disposés dans la paroi de séparation (9) autour du faisceau de membranes en fibres creuses (2) ou sur la paroi de séparation (9), sur la surface périphérique de celle-ci.

10. Module à membrane selon la revendication 1, **caractérisé en ce qu'**au moins un étranglement est réglable.

11. Module à membrane selon l'une ou plusieurs des revendications 1 à 10, **caractérisé en ce que**, vue dans la direction de l'étendue longitudinale du boîtier (1), le rapport L_{d} / Lₛ entre l'étendue L_{d} de la chambre à dialyse (11) et l'étendue Lₛ de la chambre à substituant (10) est situé entre 3 et 20.

12. Module à membrane selon la revendication 11, **caractérisé en ce que** ledit rapport est situé entre 5 et 15.

13. Module à membrane selon l'une ou plusieurs des revendications 1 à 12, **caractérisé en ce que** la paroi de séparation (9) consiste en une masse de scellement.

14. Module à membrane selon la revendication 13, **caractérisé en ce que** la paroi de séparation (9), la première et la deuxième masses de scellement (3, 4) sont réalisées avec le même matériau.

15. Module à membrane selon l'une ou plusieurs des revendications 1 à 14, **caractérisé en ce que** la paroi de séparation (9) présente une épaisseur située entre 1 et 15 mm.

16. Module à membrane selon l'une ou plusieurs des revendications 1 à 15, **caractérisé en ce que** le boîtier (1) entoure étroitement, avec sa surface intérieure, le faisceau de membranes en fibres creuses (2), dans le secteur de la chambre à dialyse (11), et présente une section transversale élargie, dans le secteur de la paroi de séparation (9) et de la chambre à substituant (10).

17. Module à membrane selon l'une ou plusieurs des revendications 1 à 16, **caractérisé en ce que**, dans le secteur de la chambre de substituant (10), un filtre stérile (28) est disposé autour du faisceau de membranes en fibres creuses (2).

18. Module à membrane selon la revendication 17, **caractérisé en ce que** le filtre stérile (28) est une membrane plate microporeuse.

19. Module à membrane selon l'une ou plusieurs des revendications 1 à 18, **caractérisé en ce que** les membranes en fibres creuses (2) présentent un taux d'ultrafiltration d'eau situé entre 20 et 1500 ml/(h.m².mmHg).

20. Module à membrane selon l'une ou plusieurs des revendications 1 à 19, **caractérisé en ce que** les membranes en fibres creuses (2) sont étanches à l'endotoxine.
